# EUROPEAN PATENT SPECIFICATION

(11) **EP 2 476 362 B1**
(45) Date of publication and mention of the grant of the patent: **14.05.2014**
(21) Application number: 11803385.1
(22) Date of filing: 06.05.2011
(51) Int. Cl.: A61B 1/00, A61B 1/06, G02B 23/24

(54) **ENDOSCOPE**
ENDOSKOP
ENDOSCOPE

(30) Priority: 08.07.2010 JP 2010156155
(43) Date of publication of application: 18.07.2012
(73) Proprietor: OLYMPUS MEDICAL SYSTEMS CORP., Tokyo 151-0072 (JP)
(72) Inventor: HONDA, Kazuki, Tokyo 151-0072 (JP); IKEDA, Yuichi, Tokyo 151-0072 (JP); KURA, Yasuhito, Tokyo 151-0072 (JP)
(74) Representative: Schicker, Silvia
(86) International application number: PCT/JP2011/060598
(87) International publication number: WO 2012/005049

(56) References cited:
- EP-A1- 2 011 428
- EP-A1- 2 027 810
- WO-A1-2006/004083
- WO-A1-2010/055800
- JP-A- 9 068 659
- JP-A- 9 313 435
- JP-A- 10 311 954
- JP-A- 11 019 028
- JP-A- 11 290 269
- JP-A- 60 021 020
- JP-A- 2002 065 589
- JP-A- 2002 112 957
- JP-A- 2003 164 418
- JP-A- 2004 216 174
- US-A1- 2010 056 869

## Description

### Technical Field

The present invention relates to an endoscope including a forward observing lens, and a forward and sideward observing dual purpose lens.

### Background Art

In recent years, endoscopes have been widely used in a medical field and an industrial field. An endoscope enables observation of an inside of a subject by insertion of an elongated insertion portion into the subject.

As endoscopes, a known direct-viewing type endoscope in which an observing lens and an illuminating lens are provided on a distal end surface of a distal end portion provided at a distal end side of an insertion portion, and a known side-viewing type endoscope in which an observing lens and an illuminating lens are provided at a part of a side surface of a distal end portion of an insertion portion are well known.

Further, in recent years, an endoscope is also well known which enables observation of not only a field of view ahead of a distal end portion of an insertion portion but also a field of view in a circumferential direction located sideward along a periphery of an outer peripheral side surface of the distal end portion at the same time.

The endoscope which enables observation of a sideward periphery in addition to a front side usually has a well-known configuration in which the endoscope has a projecting portion which projects forward from a distal end surface of a distal end portion of an insertion portion, and a forward observing lens which observes a front area is provided to face the distal end surface of the projecting portion in the projecting portion, whereas in the projecting portion, behind the forward observing lens, a lens for observing a circumferential direction is provided so that a light receiving surface faces the outer peripheral side surface in a circumferential shape along the outer peripheral side surface of the projecting portion.

Further, in the distal end portion, a lens group is located at a rear side from the circumferential direction observing lens, and, for example, an image pickup device such as a CCD is located at a condensing position of the lens group.

The configuration is such that a region to be examined which is located ahead of the distal end surface is observed by the forward observing lens, and a light which is incident on the forward observing lens passes through the circumferential direction observing lens, and forms an image in an image pick up device by the rear lens group.

Further, the configuration is such that a region to be examined which is located in the circumferential direction of the projecting portion is observed by the circumferential direction observing lens, and a light which is incident on the circumferential direction observing lens is reflected a plurality of times in the lens by a mirror or the like provided at a position at a front side of the circumferential direction observing lens, and thereafter, forms an image in the image pickup device by the rear lens group.

By the above-described configurations, not only the field of view ahead of the distal end portion of the insertion portion but also the field of view in the circumferential direction can be observed at the same time. The circumferential direction observing lens is used for forward observation as described above, and therefore, is a lens for dual purpose of forward and sideward observations.

Further, a first illuminating lens which illuminates a region to be examined which is located ahead is provided at the distal end surface of the distal end portion, and a second illuminating lens which illuminates the region to be examined located ahead with the first illuminating lens is also provided at the distal end surface of the projecting portion in order to increase light intensity distribution of the illuminating light to the region to be examined which is located ahead.

The second illuminating lens is provided at the distal end surface of the projecting portion, and a known light guide which supplies illuminating light to the second illuminating lens is inserted into the projecting portion. Thereby, the field of view in the circumferential direction of the forward and sideward observing dual purpose lens is shielded at only a region opposed to the light guide.

Further, the configuration is also well-known, in which a sideward illuminating lens which illuminates a region to be examined which is located in the circumferential direction of the projecting portion is provided at a position at a rear side from the forward and sideward observing dual purpose lens in the side surface of the projecting portion.

The fist illuminating lens and the second illuminating lens are disposed to sandwich the forward observing lens when the distal end surface of the projecting portion and the distal end surface of the distal end portion are viewed as a plane from a front.

However, in the endoscope capable of performing the observation in the circumferential direction in addition to the forward observation, there has arisen a problem that the illuminating light emitted from the first illuminating lens is incident on the light receiving surface of the forward and sideward observing dual purpose lens to cause a known incidental flare in the field of view in the circumferential direction.

Therefore, a configuration can be conceived for preventing the incidence of the illuminating light emitted from the first illuminating lens on the forward and sideward observing dual purpose lens by shortening a forward projecting length of the projecting portion so that the first illuminating lens is provided ahead of the forward observing lens. However, in this case, since the field of view of the forward observing lens is partially shielded by a member constituting the distal end portion, there has arisen a problem that the field of view in the circumferential direction is further narrowed thereby.

In view of the above problems, International Publication No. WO2006/4083 discloses the configuration in which a mirror which protrudes outward in the outside diameter of the projecting portion is provided at the forward and sideward observing dual purpose lens which is located at the side surface of the projecting portion, and part of the illuminating light emitted from the illuminating lens which is provided at the distal end surface of the distal end portion is reflected by the mirror, whereby the illuminating light emitted from the illuminating lens is prevented from being incident on the light receiving surface of the forward and sideward observing dual purpose lens while the field of view in the circumferential direction is ensured.

However, in the configuration disclosed in International Publication No. WO2006/4083, since the mirror is provided to protrude outside in a diameter direction of the projecting portion, a part of the field of view in the circumferential direction of the forward and sideward observing dual purpose lens is shielded by the mirror. Therefore, there is a problem that an irradiation range of the illuminating light which is emitted forward is restricted since the illuminating light emitted from the illuminating lens is partially shielded by the mirror as well as a problem that an image pickup range in the field of view in the circumferential direction is restricted.

The present invention has been made in view of the above problems, and an object of the invention is to provide an endoscope having a configuration capable of providing sufficient illuminating light to the region to be examined located ahead and preventing the incidence of the illuminating light on the forward and sideward observing dual purpose lens without restricting the field of view in the circumferential direction.

Document WO 2010/055800 discloses an endoscope, comprising an insertion portion, a projection portion projecting from a distal end portion of the insertion portion, a forward observing lens adapted to observe a first region, and a forward and rearward observing dual purpose lense adapted to observe a second region located reawards with regard to a peripheral side surface of the projection portion.

Document EP 2 011428 discloses an endoscope comprising a forward illuminating lens provided at a distal end surface of the endoscope, and a light shielding portion provided between the forward illuminating lens and a forward observing lens, wherein the light shielding portion comprises an inclined surface.

### Disclosure of Invention

### Means for Solving the Problem

An endoscope in one aspect of the present invention is an endoscope according to claim 1

### Brief Description of the Drawings

Fig. 1 is a view showing an outline of a configuration of an endoscope showing an embodiment;
Fig. 2 is a partial perspective view showing a distal end side of an insertion portion of the endoscope of Fig. 1 by enlargement;
Fig. 3 is a plan view of the distal end side of the insertion portion of Fig. 2, which is seen in a direction of III in Fig. 2;
Fig. 4 is a partial sectional view of the distal end side of the insertion portion taken along the IV-IV line in Fig. 3;
Fig. 5 is a view showing an observation image observed by the endoscope insertion portion of Fig. 1;
Fig. 6 is a plan view of a distal end side of an insertion portion of an endoscope in a second embodiment, which is seen from the front;
Fig. 7 is a partial sectional view of the distal end side of the insertion portion taken along the VII-VII line in Fig. 6;
Fig. 8 is a plan view of a distal end side of an insertion portion of an endoscope in a third embodiment, which is seen from the front;
   Fig. 9 is a partial sectional view of the distal end side of the insertion portion taken along the IX-IX line in Fig. 8;
   Fig. 10A is a plan view showing a modified example in which a lens at a proximal end side, which configures a first forward illuminating lens of Fig. 4 is located to be offset to an inner side in a diameter direction from a lens at a distal end side by seeing the distal end side of the insertion portion from the front;
   Fig. 10B is a partial sectional view along the 01-02-E line in Fig. 10A;
   Fig. 11 is a view showing a change of an irradiation range before and after offset of the first forward illuminating lens of Fig. 10;
   Fig. 12 is a graph showing light intensity distribution characteristics of the first forward illuminating lens before and after the offset of Fig. 10;
   Fig. 13 is a diagram showing a modified example in which the lens at the distal end side of the first forward illuminating lens of Fig. 4 is configured by two lenses with different refractive indexes, with a light intensity distribution characteristic;
   Fig. 14 is a diagram showing a modified example in which a proximal end surface of the lens at the distal end side of Fig. 4 is cut to be in a slant surface shape;
   Fig. 15 is a partial sectional view schematically showing a structure of fixing a dual purpose lens to a front side lens frame by providing a projecting portion which projects to an inner side in a diameter direction at a distal end of a rigid member, and fixing a position of the front side lens frame by butting the front side lens frame to the projecting portion;
   Fig. 16 is a partial sectional view schematically showing a structure of fixing the dual purpose lens to the front side lens frame by providing a projecting portion which projects to an inner side in the diameter direction at a distal end of a distal end cover, and fixing a position of the front side lens frame by butting the front side lens frame to the projecting portion;
Fig. 17 is a sectional view schematically showing a structure of fixing the front side lens frame by pressing a flange of the front side lens frame to the projecting portion of the rigid member of Fig. 15;
Fig. 18 is a sectional view of a rear side lens frame, a sideward illuminating lens and a fixing member of the sideward illuminating lens taken along the XVIII-XVIII line in Fig. 17;
Fig. 19 is a partial sectional view schematically showing a configuration of fixing the rear side lens frame and a resin cover to a proximal end side of the dual purpose lens without a step;
Fig. 20 is a partial sectional view schematically showing a configuration in which an ineffective field of view area is provided on a light receiving surface of the dual purpose lens;
Fig. 21A is a perspective view showing a disposition location of a nozzle which supplies a fluid to a conventional dual purpose lens, at the distal end side of the insertion portion provided with the dual purpose lens;
Fig. 21B is a top view of Fig. 21A;
Fig. 21C is a view showing an observation image which is observed by the endoscope insertion portion of Fig. 21A;
Fig. 22A is a perspective view showing a disposition location of a nozzle in a case in which a recess is provided at a region between an outer peripheral side surface of a first projecting portion and an outer peripheral side surface of a second projecting portion, at the distal end side of the insertion portion provided with the dual purpose lens;
Fig. 22B is a top view of Fig. 22A;
Fig. 22C is a view showing an observation image which is observed by the endoscope insertion portion of Fig. 22A;
Fig. 23A is a perspective view showing a disposition location of a nozzle in a case in which the nozzle is provided at an outer side in a diameter direction from Fig. 20 in the outer peripheral side surface of the second projecting portion, at the distal end side of the insertion portion provided with the dual purpose lens;
Fig. 23B is a top view of Fig. 23A;
Fig. 23C is a view showing an observation image which is observed by the endoscope insertion portion of Fig. 23A;
Fig. 24 is a view showing an observation image which is observed by a forward observing lens and the dual purpose lens;
Fig. 25 is a view showing an example in which a boundary line is provided between an area in which an image which is picked up by the forward observing lens is displayed, and an area in which an image which is picked up by the dual purpose lens of the observation image is displayed;
Fig. 26 is a partial sectional view of the insertion portion distal end side showing a configuration which forms the boundary line by making a sideward viewing angle smaller than a forward viewing angle;
Fig. 27 is a partial sectional view of the insertion portion distal end side showing a configuration which forms the boundary line by a mask;
Fig. 28 is a block diagram showing a configuration which electrically forms the boundary line in an observation image;
Fig. 29 is a view showing a display example in which an area of interest is located across an area in which an image picked up by the forward observing lens is displayed and an area in which an image picked up by the dual purpose lens is displayed, in the observation image which is observed by the forward observing lens and the dual purpose lens;
Fig. 30 is a view showing a display example in which a boundary line is provided between the area in which an image picked up by the forward observing lens is displayed and the area in which an image picked up by the dual purpose lens is displayed, and a region of interest is located across the boundary line, in the observation image of Fig. 29;
Fig. 31 is a block diagram showing a configuration which electrically deletes the boundary line from the observation image;
Fig. 32 is a partial sectional view showing an outline of a configuration of the distal end side of a conventional endoscope in the case in which an area between a sideward illuminating lens and a first forward illuminating lens in a first distal end surface is formed into a flat surface;
Fig. 33 is a partial sectional view showing an outline of a configuration of a distal end side of an endoscope in the case in which the first distal end surface is retreated rearward from that of Fig. 32; and
Fig. 34 is a partial sectional view schematically showing a state in which an area between the sideward illuminating lens and the first forward illuminating lens in the first distal end surface of Fig. 33 is formed into a slant surface shape, and an opening end portion of the first forward illuminating lens is moved forward from that of Fig. 33.

### Best Mode for Carrying Out the Invention

Hereinafter, embodiments of the present invention will be described with reference to the drawings. Attention should be paid to the fact that the drawings are schematic, and the relationship of the thickness and the width of each member, the ratios of the thicknesses of the respective members and the like differ from the actual relationship and the ratios, and the parts differing in the mutual size relationship and ratios are included among the drawings as a matter of fact.

### (First embodiment)

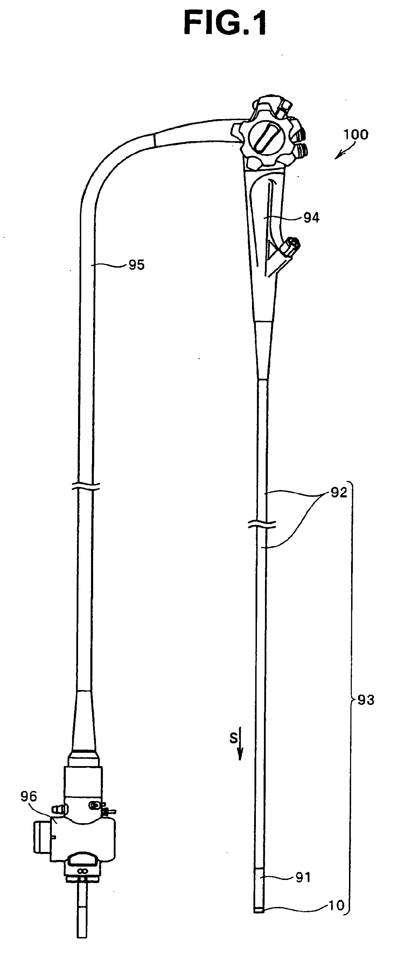

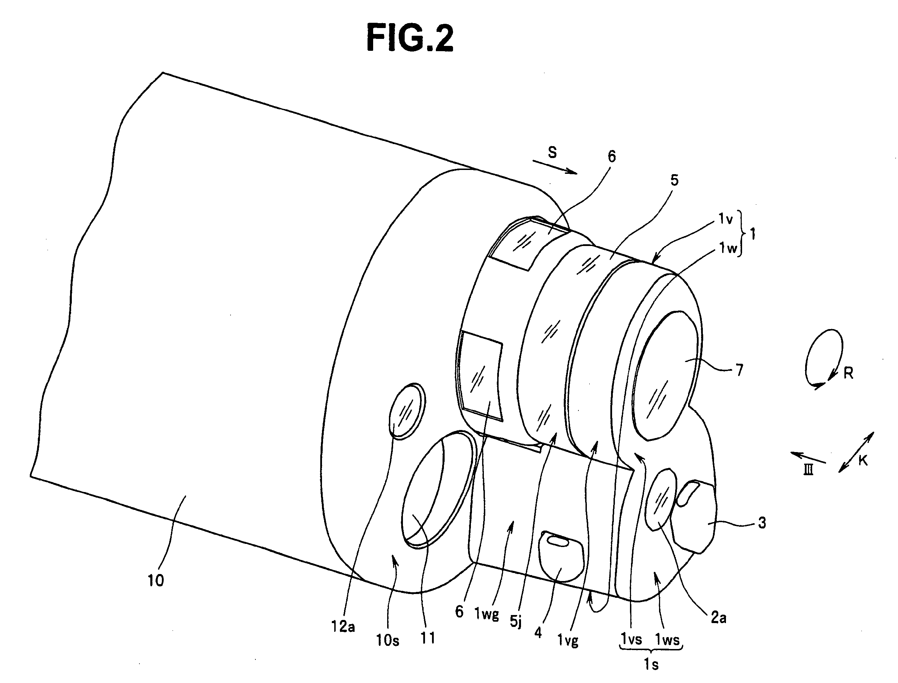

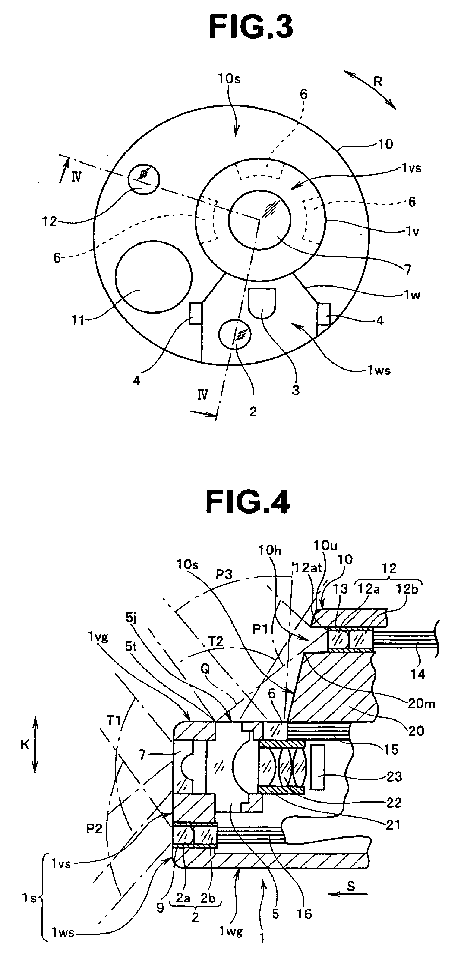

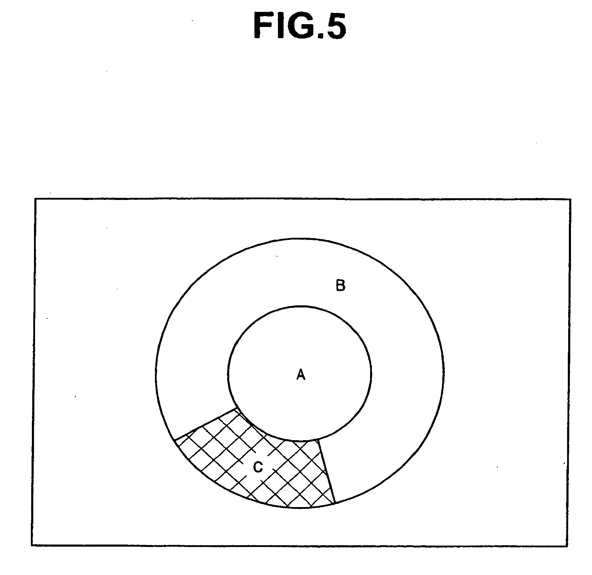

Fig. 1 is a view showing an outline of a configuration of an endoscope showing the present embodiment. Fig. 2 is a partial perspective view showing a distal end side of an insertion portion of the endoscope of Fig. 1 by enlargement. Fig. 3 is a plan view of the distal end side of the insertion portion of Fig. 2, which is seen in the direction of III in Fig. 2. Fig. 4 is a partial sectional view of the distal end side of the insertion portion taken along the IV-IV line in Fig. 3. Fig. 5 is a view showing an observation image which is observed by the endoscope insertion portion of Fig. 1.

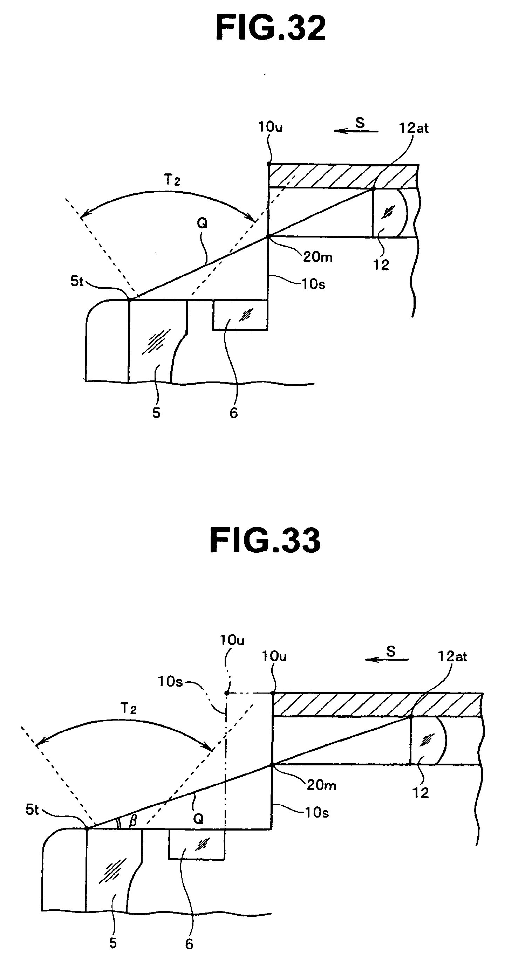

Further, Fig. 32 is a partial sectional view showing an outline of a configuration of the distal end side of a conventional endoscope in the case in which an area between a sideward illuminating lens and a first forward illuminating lens in a first distal end surface is formed into a flat surface.

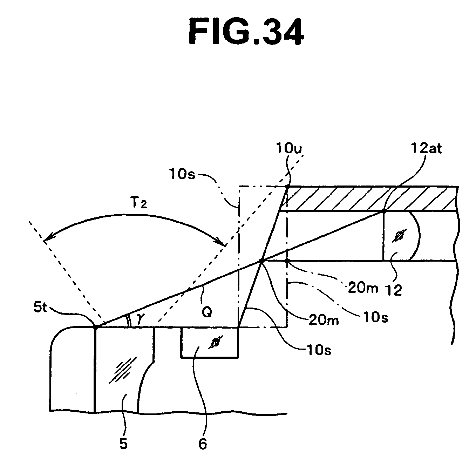

Further, Fig. 33 is a partial sectional view showing an outline of a configuration of a distal end side of an endoscope in the case in which the first distal end surface is retreated rearward from that of Fig. 32. Fig. 34 is a partial sectional view schematically showing a state in which an area between the sideward illuminating lens and the first forward illuminating lens in the first distal end surface of Fig. 33 is formed into a slant surface shape, and an opening end portion of the first forward illuminating lens is moved forward from that of Fig. 33.

As shown in Fig. 1, an endoscope 100 has a main part configured by including an insertion portion 93 which is inserted into a subject, an operation portion 94 which is provided at a proximal end side in an insertion direction S (hereinafter, simply called a proximal end side) of the insertion portion 93, a universal cord 95 which is extended from the operation portion 94, and a connector 96 which is provided at an extended end of the universal cord 95.

The connector 96 is connectable to a known light source apparatus or the like, which is not illustrated, and thereby, the endoscope 100 is connectable to peripheral apparatuses.

The insertion portion 93 has a main part configured by including a distal end portion 10, a bending portion 91 and a flexible tube portion 92 in sequence from a distal end side in the insertion direction S (hereinafter, simply called a distal end side).

As shown in Figs. 2 to 4, a projecting portion 1 having a sectional shape of substantially a figure of 8 projects forward in the insertion direction S (hereinafter, simply called forward) from a first distal end surface 10s at the distal end side of the distal end portion 10. The projecting portion 1 may be formed integrally with the distal end portion 10, or may be formed to be a separate piece.

The projecting portion 1 is formed to be substantially 8-shaped in section by a first projecting portion 1v having a substantially columnar shape, and a second projecting portion 1w having a polygonal column shape and located side by side with respect to the first projecting portion 1v in a diameter direction K of the distal end portion 10.

A forward observing lens 7 which is located at a front side from a distal end surface 1vs and observes a first region to be examined of a subject is provided by being fixed to a rigid member 20 provided in the first projecting portion 1v so as to face a second distal end surface 1s at the distal end side of the projecting portion 1, more specifically, the distal end surface 1vs which is the second distal end surface at the distal end side of the first projecting portion 1v. The forward observing lens 7 has a viewing angle with an observation range T1 as shown in Fig. 4.

Further, in the second projecting portion 1w, a second forward illuminating lens 2 is provided so that a lens 2a located at the distal end side, of the second forward illuminating lens 2, faces a distal end surface 1ws which is a second distal end surface at a distal end side of the second projecting portion 1w. The second forward illuminating lens 2 has an irradiation range P2 as shown in Fig. 4 and irradiates the first region to be examined with illuminating light.

As shown in Fig. 4, the second forward illuminating lens 2 is fixed to the rigid member 20 which is provided in the second projecting portion 1w in a state in which the lens 2a and a lens 2b which is located at a proximal end side from the lens 2a are held by a lens frame 9. The second forward illuminating lens 2 may be configured by one lens or may be configured by three lenses or more.

A distal end surface of a light guide 16, which is inserted into insides of the connector 96, the universal cord 95, the operation portion 94 and the insertion portion 93, is located opposite a proximal end surface of the lens 2b of the second forward illuminating lens 2.

As a result, when the connector 96 is connected to the light source apparatus, and illuminating light is supplied from the light source apparatus, the illuminating light is supplied to the second forward illuminating lens 2 through the light guide 16, and is expanded and emitted to the first region to be examined, by the second forward illuminating lens 2.

Further, as shown in Figs. 2 and 3, in the distal end surface 1ws of the second projecting portion 1w, a nozzle 3 which cleans the forward observing lens 7 by supplying a fluid to the forward observing lens 7 is provided in the vicinity of the lens 2a of the second forward illuminating lens 2.

Further, in the first projecting portion 1v, at a rear side in the insertion direction S (hereinafter, simply called a rear side) from the forward observing lens 7, a forward and sideward observing dual purpose lens having a columnar shape (hereinafter, simply called a dual purpose lens) 5 is provided by being fixed to the rigid member 20 which is provided in the first projecting portion 1v so that a light receiving surface 5j faces the outer peripheral side surface 1vg of the first projecting portion 1v along a circumferential direction R, that is, so that the light receiving surface 5j is exposed to the outer peripheral side surface 1vg.

The dual purpose lens 5 observes the first region to be examined with the forward observing lens 7, and also observes a second region to be examined of the subject which is located opposite a circumference of the outer peripheral side surface 1vg of the first projecting portion 1v. More specifically, the dual purpose lens 5 has the field of view in the circumferential direction described above. The dual purpose lens 5 has a viewing angle of an observation range T2 as shown in Fig. 4.

Further, the light receiving surface 5j of the dual purpose lens 5 is exposed only on the outer peripheral side surface 1vg of the first projecting portion 1v, and therefore, is not exposed onto the outer peripheral side surface 1wg of the second projecting portion 1w. Further, in the region where the first projecting portion 1v is joined to the second projecting portion 1w, the light receiving surface 5j is hidden by the second projecting portion 1w, and therefore, is not exposed onto the outer peripheral side surface 1vg.

Further, as shown in Figs. 2 and 3, the dual purpose lens 5 is cleaned by nozzles 4 a plurality of which are provided at the outer peripheral side surface 1wg of the second projecting portion 1w, and which supply a fluid to the dual purpose lens 5.

As shown in Figs. 2 to 4, in the first projecting portion 1v, a plurality of sideward illuminating lenses 6 which illuminate the second region to be examined are provided at a rear side from the dual purpose lens 5 by being fixed to the rigid member 20 in the first projecting portion 1v so as to face the outer peripheral side surface 1vg. The side illuminating lens 6 has an irradiation range P3 and irradiates the second region to be examined with illuminating light, as shown in Fig. 4.

At the proximal end surface of the sideward illuminating lens 6, a distal end surface of a light guide 15, which is inserted into the insides of the connector 96, the universal cord 95, the operation portion 94 and the insertion portion 93, is located opposite the proximal end surface of the sideward illuminating lens 6.

Accordingly, when the connector 96 is connected to the light source apparatus, and illuminating light is supplied from the light source apparatus, the illuminating light is supplied to the sideward illuminating lens 6 through the light guide 15, and is expanded and irradiates the second region to be examines by the sideward illuminating lens 6.

Further, in the first projecting portion 1v, a lens frame 21 which holds a plurality of lenses 22 is fixed to an image formation position of the dual purpose lens 5 in the rear side from the dual purpose lens 5, and an image pickup device 23 such as a CCD is fixed to an image formation position of the lens 22. The lens 22 and the image pickup device 23 may be provided in the distal end portion 10.

Thereby, light is incident on the forward observing lens 7, and the image of the first region to be examined is formed in the image pickup device 23 through the dual purpose lens 5 and the lens 22. As a result, the image of the first region to be examined is displayed on the monitor as a circular area A as shown in Fig. 5.

Further, as for an image of the second region to be examined, light is incident on the dual purpose lens 5 through the light receiving surface 5j, and is reflected a plurality of times by the mirror and the like, which are not illustrated, provided at the dual purpose lens 5, and thereafter, the image of the second region to be examined is formed in the image pickup device 23 through the lens 22. As a result, the image is displayed as an annular area B on the outer circumference of the circular area A on the monitor as shown in Fig. 5.

The image of the second region to be examined does not become completely circular as shown in the area B on the monitor, and only an area C is omitted and is not displayed. This is because as described above, in the light receiving surface 5j of the dual purpose lens 5 which is exposed on the outer peripheral side surface 1vg of the first projecting portion 1v, the region of the first projecting portion 1v, which is joined to the second projecting portion 1w is hidden by the second projecting portion 1w.

Further, to a first distal end surface 10s of the distal end portion 10, an opening 11 of a known treatment instrument insertion tube which is provided in the insertion portion 93 is opened.

Further, in the distal end portion 10, a lens 12a which is located at a distal end side of a first forward illuminating lens 12 which illuminates the first region to be examined with the second forward illuminating lens 2 is provided to face the first distal end surface 10s. The first forward illuminating lens 12 has an irradiation range P1 and irradiates the first region to be examined with illuminating light as shown in Fig. 4.

As shown in Fig. 4, the first forward illuminating lens 12 is fixed to the rigid member 20 which is provided in the distal end portion 10 in a state in which the lens 12a and a lens 12b which is located coaxially with the lens 12a at a proximal end side from the lens 12a are held by a lens frame 13.

More specifically, in the rigid member 20, the lens frame 13 which holds the lens 12a and the lens 12b is fitted in and fixed to a fit hole 10h which is formed along the insertion direction S and has an opening in the first distal end surface 10s, whereby the first forward illuminating lens 12 is provided in the distal end portion 10.

The first forward illuminating lens 12 may be configured by one lens, or may be configured by three lenses or more.

Here, as shown in Fig. 4, an opening end portion 20m which is located at an inner side in a diameter direction K, of the fit hole 10h is located on a phantom line Q which linearly connects an end portion 5t at the distal end side of the light receiving surface 5j of the dual purpose lens 5 which faces the outer peripheral side surface 1vg of the first projecting portion 1v, and an end portion 12sat at an outer side in the diameter direction K, of the lens 12a in the first forward illuminating lens 12, and thereby, the illuminating light which is emitted from the first forward illuminating lens 12 is prevented from being incident on the dual purpose lens 5.

In other words, the lens 12a is located at a rear side from the phantom line Q, and thereby, the illuminating light which is emitted from the first forward illuminating lens 12 is prevented from being incident on the dual purpose lens 5 by the opening end portion 20m.

This is because the illuminating light emitted from the first forward illuminating lens 12 is shielded by the opening end portion 20m. Accordingly, in the present embodiment, the opening end portion 20m configures a light shielding portion.

Further, the opening end portion 20m is located so as not to protrude into the aforementioned irradiation range P1 of the first forward illuminating lens 12, and thereby, does not narrow the irradiation range P1 of the first forward illuminating lens 12.

Furthermore, the opening end portion 20m is located in the area outside the observation range T2 of the dual purpose lens 5, and thereby, the first forward illuminating lens 12 does not enter the inside the observation range T2 of the dual purpose lens 5.

Further, in the first distal end surface 10s of the distal end portion 10, in an area between the sideward illuminating lens 6 and the first forward illuminating lens 12, the opening end portion 20m is formed to have an inclined surface which inclines to be located at a rear side from the sideward illuminating lens 6, from the sideward illuminating lens 6 which is located at an inner side in the diameter direction K toward the first forward illuminating lens 12 which is located at an outer side in the diameter direction K, as shown in Figs. 3 and 4.

This is because if the area between the sideward illuminating lens 6 and the first forward illuminating lens 12 in the first distal end surface 10s is formed into a flat surface, an outer peripheral edge portion 10u of the distal end portion 10 enters the observation range T2 of the dual purpose lens 5 as shown in Fig. 32. For this reason, usually, as shown in Fig. 33, the first distal end surface 10s is generally located to be retreated rearward as the first distal end surface 10s shown by the solid line of Fig. 33 from the first distal end surface 10s shown by the chain double-dashed line of Fig. 33 which corresponds to the first distal end surface 10s of Fig. 32, so that the outer peripheral edge portion 10u does not enter the inside the observation range T2.

However, in the configuration shown in Fig. 33, the problem arises, that the rigid member 20 becomes long in the insertion direction S correspondingly to the amount by which the first distal end surface 10s is retreated rearward, and operability of the endoscope is reduced.

Thus, as shown in Fig. 34 and Fig. 4, the inclined surface is formed in the area between the sideward illuminating lens 6 and the first forward illuminating lens 12 in the first distal end surface 10s. According thereto, an angle which is formed by the outer peripheral side surface 1vg and the phantom line Q at the end portion 5t can be made larger than in the case in which the region between the sideward illuminating lens 6 and the first forward illuminating lens 12 in the first distal end surface 10s is formed into a flat surface shown in Fig. 33 (β < γ).

Accordingly, as shown in Fig. 34, the opening end portion 20m which shields illuminating light can be located at a front side from the opening end portion 20m of Fig. 33 which is shown by the dashed line in Fig. 34, while the outer peripheral edge portion 10u remains not to enter the inside the observation range T2, and therefore, the first forward illuminating lens 12 can be located at the front side.

From the above description, the rigid length in the insertion direction S of the rigid member 20 which is provided in the projecting portion 1 and the distal end portion 10 can be made short. The area is not limited to the inclined surface, and even if the area is formed into the shape inclined stepwise from the sideward illuminating lens 6 towards the first forward illuminating lens 12, the same effect can be obtained.

Further, in the first distal end surface 10s, the area between the first forward illuminating lens 12 and the outer peripheral edge portion of the distal end portion 10 is also preferably formed into an inclined surface as shown in Fig. 4. This is because if the area is not formed into the inclined surface, the area enters the observation range T2 of the dual purpose lens 5.

As described above, in the present embodiment, the opening end portion 20m which is located at the inner side in the diameter direction K, of the fit hole 10h formed along the insertion direction S is shown to be located on the phantom line Q which linearly connects the end portion 5t at the distal end side of the light receiving surface 5j of the dual purpose lens 5 which faces the outer peripheral side surface 1vg of the first projecting portion 1v, and the end portion 12at at the outer side in the diameter direction K, of the lens 12a in the first forward illuminating lens 12, in the first distal end surface 10s of the distal end portion 10.

According to the above, the illuminating light emitted from the first forward illuminating lens 12 is shielded by the opening end portion 20m, and therefore, the illuminating light emitted from the first forward illuminating lens 12 is prevented from being incident on the light receiving surface 5j of the dual purpose lens 5 by the opening end portion 20m. Therefore, occurrence of an incidental flare to the observation image picked up by the dual purpose lens 5 in the area B of Fig. 5 can be prevented.

Further, the opening end portion 20m is located outside the observation range T2 of the dual purpose lens 5, and therefore, the observation range of the dual purpose lens 5 is not limited.

Furthermore, the opening end portion 20m is located not to protrude into the irradiation range P1 of the first forward illuminating lens 12, and therefore, does not restrict the irradiation range P1.

From the above description, the endoscope 100 can be provided, which has the configuration that can supply sufficient illuminating light to the region to be examined ahead, does not limit the field of view in the circumferential direction, and can prevent entry of the illuminating light into the dual purpose lens 5.

### (Second embodiment)

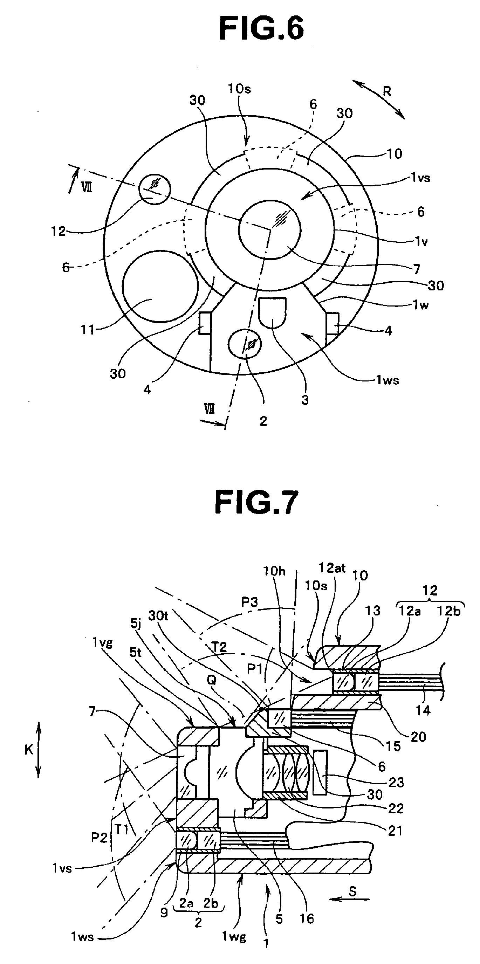

Fig. 6 is a plan view of a distal end side of an insertion portion of an endoscope in the present embodiment, which is seen from the front, and Fig. 7 is a partial sectional view of the distal end side of the insertion portion taken along the VII-VII line in Fig. 6.

A configuration of the endoscope of the second embodiment differs from the endoscope of the first embodiment shown in Figs. 1 to 5 described above in that a light shielding portion is formed at a lens frame which holds a sideward illuminating lens. Accordingly, only the difference will be described, and the same components as in the first embodiment are assigned with the same reference numerals and characters, and the description thereof will be omitted.

As shown in Figs. 6 and 7, in the present embodiment, the sideward illuminating lens 6 is located to protrude to an outer side in the diameter direction K, from the outer peripheral side surface 1vg by being held by a lens frame 30 provided in the first projecting portion 1v.

Further, an end portion 30t which is located at an outer side in the diameter direction K, of the lens frame 30 is located on the aforementioned phantom line Q which linearly connects the end portion 5t at the distal end side of the light receiving surface 5j of the dual purpose lens 5 which faces the outer peripheral side surface 1vg of the first projecting portion 1v, and the end portion 12at at the outer side in the diameter direction K, of the lens 12a in the first forward illuminating lens 12, and thereby, the illuminating light emitted from the first forward illuminating lens 12 is prevented from being incident on the dual purpose lens 5.

In other words, the lens 12a is located at a rear side from the phantom line Q, and therefore, the illuminating light emitted from the first forward illuminating lens 12 is prevented from being incident on the dual purpose lens 5 by the end portion 30t. This is because the illuminating light emitted from the first forward illuminating lens 12 is shielded by the end portion 30t. Accordingly, in the present embodiment, the end portion 30t configures the light shielding portion.

Further, the end portion 30t is located not to protrude into the aforementioned irradiation range P1 of the first forward illuminating lens 12, and thereby, the end portion 30t is prevented from restricting the irradiation range P1 of the first forward illuminating lens 12.

From the above description, the distal end side of the insertion portion 93 is formed into the shape which is inclined stepwise from the dual purpose lens 5 toward the first forward illuminating lens 12, by the lens frame 30.

According to the configuration like this, the same effect as in the aforementioned first embodiment can also be obtained, in addition to which, a space in the diameter direction K between the sideward illuminating lens 6 and the first forward illuminating lens 12 can be made smaller than in the first embodiment by the configuration in which the illuminating light emitted from the first forward illuminating lens 12 is shielded by the end portion 30t of the lens frame 30.

In other words, the first forward illuminating lens 12 can be located at the sideward illuminating lens 6 side in the diameter direction K, and therefore, the diameter at the distal end side of the insertion portion 93 can be reduced more than in the first embodiment.

### (Third embodiment)

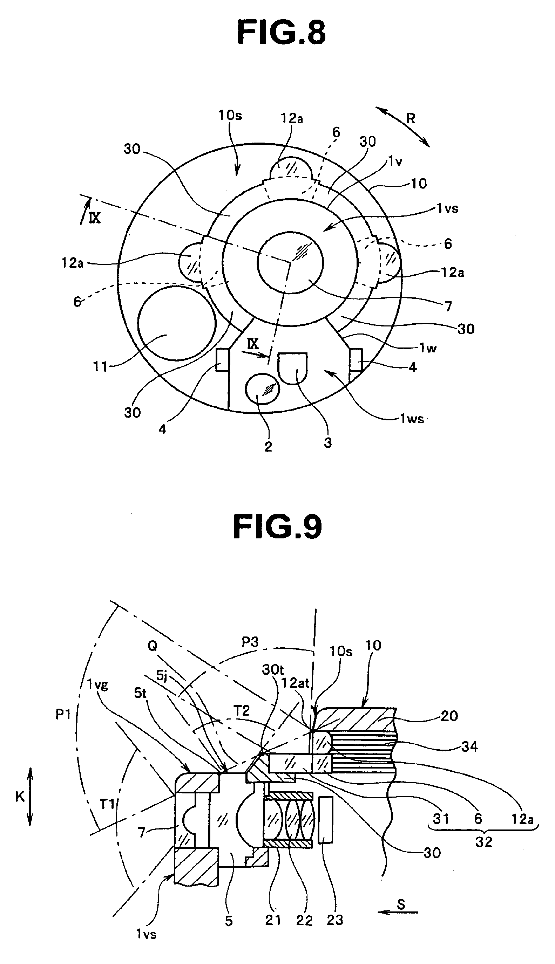

Fig. 8 is a plan view of a distal end side of an insertion portion of an endoscope in the present embodiment, which is seen from the front, and Fig. 9 is a partial sectional view of the distal end side of the insertion portion taken along the IX-IX line in Fig. 8.

A configuration of the endoscope of the third embodiment differs from the endoscope of the second embodiment shown in Figs. 6 and 7 described above in that a sideward illuminating lens and a first forward illuminating lens are integrally formed. Accordingly, only the difference will be described, and the same components as in the second embodiment are assigned with the same reference numerals and characters, and the description thereof will be omitted.

As shown in Figs. 8 and 9, in the present embodiment, the sideward illuminating lens 6 and the distal end lens 12a of the first forward illuminating lens 12 are integrally formed as a forward and sideward dual purpose illumination unit 32 with use of a light-guiding plate 31.

In the above configuration, the sideward illuminating lens 6 emits illuminating light toward a front side in the insertion direction S, and the irradiation direction of the illuminating light is configured to be changed toward the second region to be examined by a light-guiding plate 31.

Consequently, one light guide 34 can be used as both the light guide 14 for the first forward illuminating lens 12, and the light guide 15 of the sideward illuminating lens 6.

The light-guiding plate 31 is held by the lens frame 30 provided in the first projecting portion 1v. The light-guiding plate 31 is located to protrude to an outer side in the diameter direction K from the outer peripheral side surface 1vg by being held by the lens frame 30.

Further, in the present embodiment, the end portion 30t which is located at the outer side in the diameter direction K of the lens frame 30 is also located on the aforementioned phantom line Q which linearly connects the end portion 5t at the distal end side of the light receiving surface 5j of the dual purpose lens 5 which faces the outer peripheral side surface 1vg of the first projecting portion 1v, and the end portion 12at at the outer side in the diameter direction K of the lens 12a in the first forward illuminating lens 12, and thereby, the illuminating light emitted from the first forward illuminating lens 12 is prevented from being incident on the dual purpose lens 5.

According to the configuration like this, the same effect as in the aforementioned second embodiment can also be obtained, in addition to which, the first forward illuminating light guide and the sideward illuminating light guide are configured from one light guide, and therefore, a space in the diameter direction K between the sideward illuminating lens 6 and the first forward illuminating lens 12 can be eliminated.

In other words, the first forward illuminating lens 12 can be located nearer to the sideward illuminating lens 6 side in the diameter direction K than in the second embodiment, and therefore, the diameter at the distal end side of the insertion portion 93 can be reduced more than in the second embodiment.

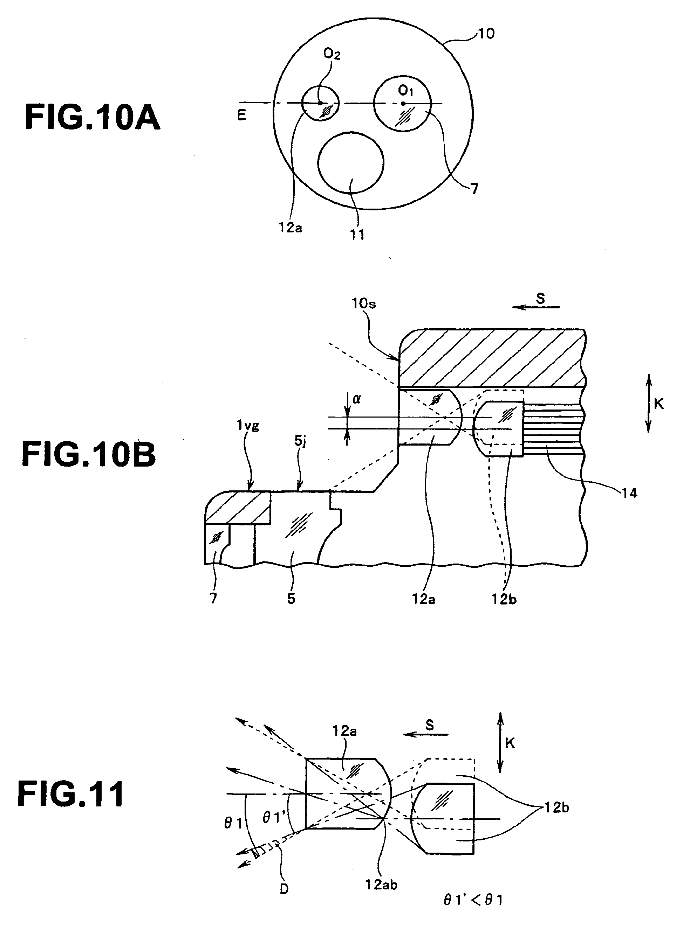

Hereinafter, a modified example will be shown with use of Figs. 10 to 12. Fig. 10A is a plan view showing a modified example in which the lens at the proximal end side which configures the first forward illuminating lens of Fig. 4 is located to be offset to an inner side in the diameter direction from the lens at the distal end side, by seeing the distal end side of the insertion portion from the front, and Fig. 10B is a partial sectional view taken along the 01-02-E line in Fig. 10A.

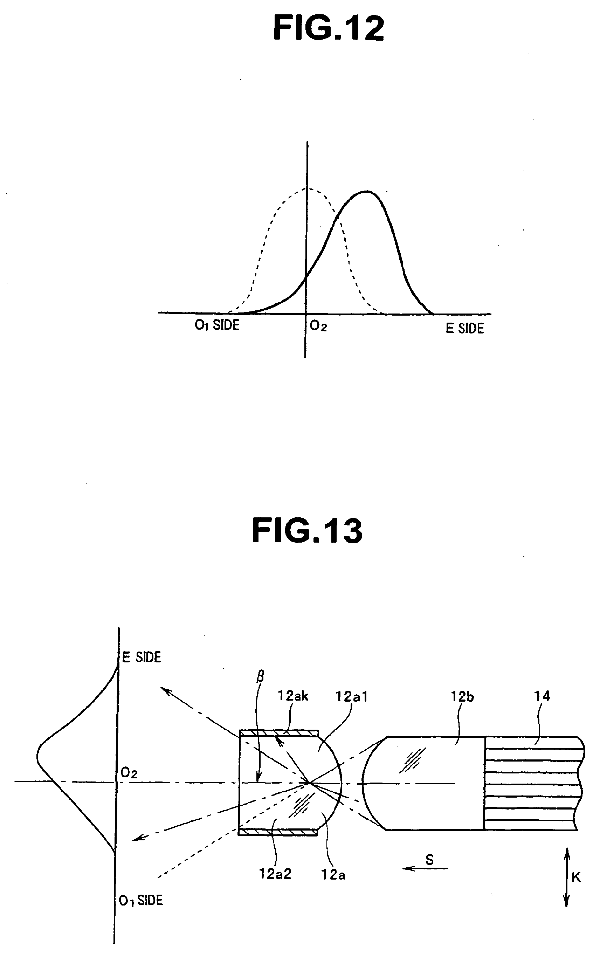

Further, Fig. 11 is a view showing a change of an irradiation range before and after the offset of the first forward illuminating lens of Fig. 10, and Fig. 12 is a graph showing light intensity distribution characteristics of the first forward illuminating lens before and after the offset of Fig. 10.

In the aforementioned first to third embodiments, it is shown that the first forward illuminating lens 12 is configured by the two lenses 12a and 12b. Further, as shown in Fig. 4, it is shown that the lens 12a and the lens 12b are coaxially disposed.

However, the present invention is not limited to the above description, and the lens 12b may be provided to be offset by α to the inner side in the diameter direction K, that is, the side where the image pickup device 23 is provided, as shown in Figs. 10A and 10B.

According to the above, an illuminating light emitted from the lens 12b after being offset is refracted in a proximal end surface 12ab of the lens 12a, and therefore, a light intensity distribution peak of the illuminating light emitted from the lens 12a shifts as shown in Fig. 11. For this reason, an irradiation angle θ1' from the lens 12a of the illuminating light which is emitted in the inner side direction in the diameter direction K becomes smaller than an irradiation angle θ1 before the offset (θ1' < θ1), and therefore, the components of the illuminating light emitted to the inner side in the diameter direction K can be cut by the amount of the oblique lines shown in Fig. 11.

Further, the light intensity distribution characteristic of the first forward illuminating lens 12 is set to have the light intensity distribution peak in the center, but the light intensity distribution peak can be shifted to an E side, that is, an outer side in the diameter direction K of the distal end portion 10 from the light intensity distribution peak before the offset as shown by the solid line, as shown in Fig. 12.

From the above description, when the lens 12b is disposed to be offset to the inner side in the diameter direction with respect to the lens 12a, the illuminating light which is emitted from the first forward illuminating lens 12 has the irradiation range of irradiation to the dual purpose lens 5 side restricted, and the light intensity distribution peak shifts to the outer side in the diameter direction K, and therefore, the illuminating light can be more prevented from being incident on the dual purpose lens 5, in addition to the effects of the configurations of the first embodiment to the third embodiment.

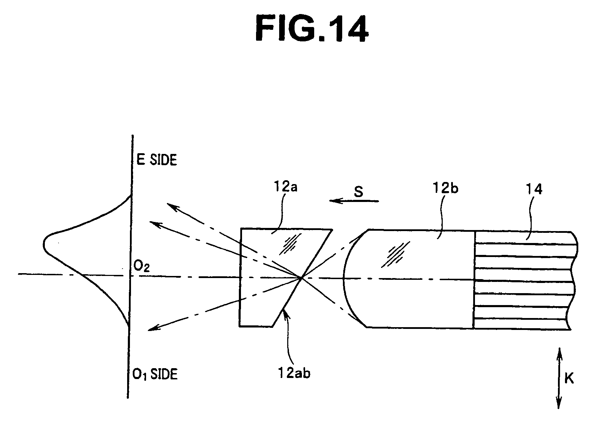

Hereinafter, other modified examples will be shown with use of Figs. 13 and 14. Fig. 13 is a diagram showing a modified example in which the lens at the distal end side of the first forward illuminating lens of Fig. 4 is configured by two lenses with different refractive indexes, with the light intensity distribution characteristic, and Fig. 14 is a diagram showing a modified example in which a proximal end surface of the lens at the distal end side of Fig. 4 is cut to be in a slant surface shape.

As shown in Fig. 13, when in the lens 12a at the distal end side of the first forward illuminating lens 12, a lens with a high refraction index is used for a lens 12a1 at an outer side in the diameter direction, that is, at the outer peripheral edge portion side of the distal end portion 10 than a lens 12a2 at an inner side in the diameter direction, with respect to a center axis, that is, at the image pickup device 23 side, and a light absorber 12ak is formed on an outer peripheral surface of each of the lenses 12a1 and 12a2 by coating or the like, a light beam which passes to the lens 12a2 side comes out to a 02 side by refraction at a β surface, and thereby, the components of the light beams which pass to the lens 12a2 side are substantially cut.

Further, due to the difference in the refraction index of the lens 12a1 and the lens 12a2, the light intensity distribution peak is shifted to the E side similarly to the configuration of Fig. 10 to Fig. 12. Therefore, the component of the light which is incident on the dual purpose lens 5 can be reduced, and if the configuration of the modified example is used with the configurations of the first embodiment to the third embodiment, light incident on the dual purpose lens 5 can be prevented more effectively.

Even if the proximal end surface 12ab of the lens 12a is cut to be in a slant surface shape as shown in Fig. 14 in the above description, the same effect as in Fig. 13 can be obtained.

Incidentally, in the configuration including the forward observing lens 7, the dual purpose lens 5 and a plurality of lenses 22 described above, each of the lenses 7 and 5 is usually held in the lens frame provided in the rigid member 20 in general, though not illustrated in the aforementioned first to third embodiments.

However, the dual purpose lens 5 has the configuration in which the second region to be examined with the circumferential light receiving surface 5j facing the outer peripheral side surface 1vg is observed, and therefore, if the dual purpose lens 5 is put into the lens frame, the light receiving surface 5j is blocked by the lens frame.

Further, the dual purpose lens 5 has the outside diameter in the diameter direction K formed to be larger than the forward observing lens 7, and therefore, even if the region opposed to the light receiving surface 5j, of the lens frame is cut out, it is difficult in assembly to fix the forward observing lens 7, the dual purpose lens 5 and the lens 22 into one lens frame.

Consequently, the dual purpose lens 5 is conventionally sandwiched longitudinally along the insertion direction S with use of the lens frame at the front side, which holds the forward observing lens 7, and the lens frame at the rear side which holds the lens 22, and the dual purpose lens 5 is bonded to the front side lens frame and the rear side lens frame, whereby the dual purpose lens 5 is fixed to the two lens frames.

However, in the above fixing structure, the dual purpose lens 5 is provided inside the first projecting portion 1v in addition to that the fixing force is weaker than the configuration in which various lenses to one lens frame are fixed, and therefore, a problem arises, that the first projecting portion 1v is more susceptible to an external force than ordinary direct-viewing type endoscopes and side-viewing type endoscopes, and once the projecting portion receives the external force, the dual purpose lens 5 easily falls off from the front side lens frame and the rear side lens frame.

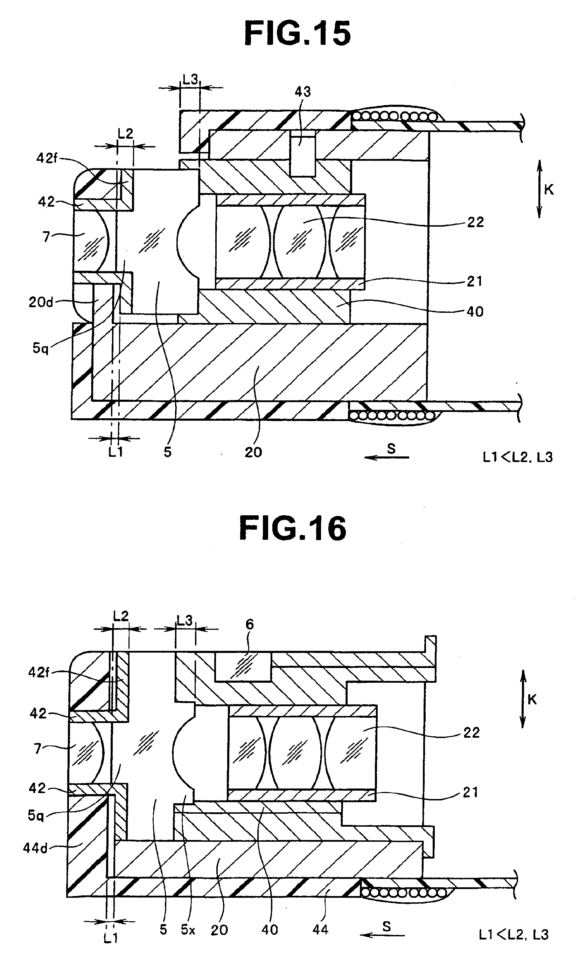

Hereinafter, configurations that solve the problem like this will be shown with use of Figs. 15 to 18. Fig. 15 is a partial sectional view schematically showing a structure which fixes the dual purpose lens to the front side lens frame by providing a projecting portion which projects to an inner side in the diameter direction at the distal end of the rigid member, and fixing a position of the front side lens frame by butting the front side lens frame to the projecting portion.

Further, Fig. 16 is a partial sectional view schematically showing a structure which fixes the dual purpose lens to the front side lens frame by providing a projecting portion which projects to an inner side in the diameter direction at a distal end of a distal end cover, and fixing a position of the front side lens frame by butting the front side lens frame to the projecting portion.

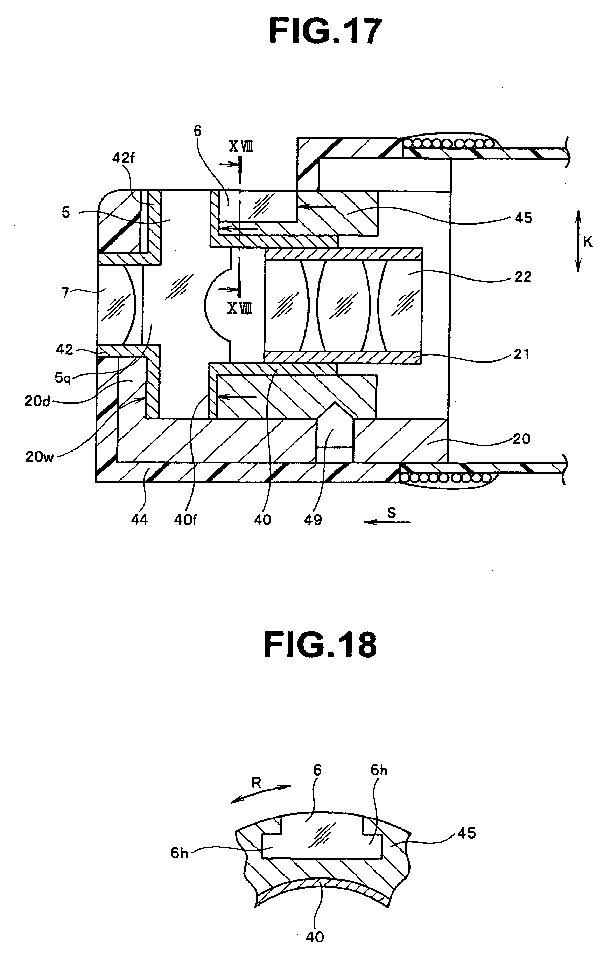

Further, Fig. 17 is a sectional view schematically showing a structure which fixes the front side lens frame by pressing a flange of the front side lens frame to the projecting portion of the rigid member of Fig. 15. Fig. 18 is a sectional view of a rear side lens frame, a sideward illuminating lens and a fixing member of the sideward illuminating lens taken along the XVIII-XVIII line in Fig. 17.

As shown in Fig. 15, in the first projecting portion 1v, the forward observing lens 7 is held in a front side lens frame 42 which is provided in the rigid member 20, and a projecting portion 5q which projects forward to the dual purpose lens 5 is fitted in and fixed to the front side lens frame 42 by, for example, bonding, with a fitting length L2 along the insertion direction S.

Further, a rear side lens frame 40 which is provided in the rigid member 20 is fitted on and fixed to an outer periphery at the proximal end side of the dual purpose lens 5 by, for example, bonding with a fitting length L3 along the insertion direction S, and a plurality of lenses 22 are fixed to the lens frame 21 which is provided in the rear side lens frame 40. The rear side lens frame 40 is fixed to the rigid member 20 with a fixing member 43.

Here, in the present configuration, a projecting portion 20d which projects toward an inner side of the diameter direction K, more specifically, the forward observing lens 7 side is provided at the distal end side of the rigid member 20.

A projecting end of the projecting portion 20d is butted to an outer peripheral surface of the front side lens frame 42. Further, a space of L1 is formed along the insertion direction S, between the projecting portion 20d and a flange 42f which is formed at a proximal end side of the front side lens frame 42. The space L1 is set to be smaller than the fitting lengths L2 and L3.

According to the configuration like this, even if bonding of the dual purpose lens 5 breaks away with respect to the front side lens frame 42 and the rear side lens frame 40, the front side lens frame 42 is restrained from moving in the insertion direction S by the projecting portion 20d, and therefore, fitting of the projecting portion 5q of the dual purpose lens 5 to the front side lens frame 42 is maintained.

Accordingly, the dual purpose lens 5 can be reliably prevented from slipping off from the front side lens frame 42.

The above configuration may be realized by using a projecting portion 44d of a distal end cover 44 with which an outer periphery of the rigid member 20 is covered, without being limited to the projecting portion 20d of the rigid member 20.

More specifically, as shown in Fig. 16, the projecting portion 44d which projects toward the inner side in the diameter direction K, more specifically, the forward observing lens 7 side is provided at a distal end side of the distal end cover 44 with which the outer periphery of the rigid member 20 is covered. In the present configuration, the projecting portion 20d is not provided at the distal end of the rigid member 20.

Further, a projected end of the projecting portion 44d is butted to the outer peripheral surface of the front side lens frame 42. Further, the space of L1 is formed along the insertion direction S between the projecting portion 44d and the flange 42f of the front side lens frame 42. The space L1 is set to be in a dimension smaller than the fitting lengths L2 and L3 as in Fig. 15.

According to the configuration like this, even if bonding of the dual purpose lens 5 breaks away with respect to the front side lens frame 42 and the rear side lens frame 40, the front side lens frame 42 is restrained from moving in the insertion direction S by the projecting portion 44d, and thereby, fitting of the projecting portion 5q of the dual purpose lens 5 to the front side lens frame 42 is maintained.

Accordingly, the dual purpose lens 5 can be reliably prevented from slipping off from the front side lens frame 42.

In the configuration shown in Fig. 16, a stepped portion 5x is formed on a rear end surface of the dual purpose lens 5, and the rear side lens frame 40 is configured to be fitted to the stepped portion 5x. According to the configuration like this, the distal end side of the insertion portion 93 can be reduced in diameter more than in the configuration shown in Fig. 15.

Further, as another fixing structure, the structure may be used, which is such that a screw 49 is fastened to a fixing member 45 of the sideward illuminating lens 6, which is fixed to the outer periphery of the rear side lens frame 40 from the outer side in the diameter direction K, and thereby, the fixing member 45 moves forward in the insertion direction by surface contact of inclined surfaces formed on a receiving surface of the fixing member 45 and inclined surfaces formed on a distal end surface of the screw 49, whereby the fixing member 45 is pressed against the sideward illuminating lens 6 and the flange 40f of the rear side lens frame 40, and the flange 42f of the front side lens frame 42 is further pressed against the aforementioned projecting portion 20d of the rigid member 20 as shown in Fig. 17.

According to the above description, a compression force occurs along the insertion direction S on the bonding surfaces of the dual purpose lens 5 to the front side lens frame 42 and the rear side lens frame 40, whereby the bonding force of the dual purpose lens 5 to the front side lens frame 42 and the rear side lens frame 40 is reinforced, and therefore, by the simple configuration that uses the screw 49, the number of components can be reduced, in addition to that fixing of the dual purpose lens 5 can be made strong.

Further, if the configuration which fixes the sideward illuminating lens 6 to the fixing member 45 by a hook portion 6h as shown in Fig. 18 is adopted, the sideward illuminating lens 6 also can be prevented from falling off.

Incidentally, as described above, the dual purpose lens 5 is fixed by being sandwiched by the front side lens frame and the rear side lens frame in the projecting portion which projects forward from the distal end portion of the insertion portion. Further, the configuration is well-known, in which in order to keep the insulation property of each of the lens frames, the outer periphery of each of the lens frames is covered with a resin cover.

However, the rear side lens frame is usually fitted to the outer periphery of the proximal end side of the dual purpose lens 5, and therefore, a problem arises, that mucus, dust and the like easily accumulate in corner portions of a step between the light receiving surface 5j of the dual purpose lens 5 and a fitting region of the rear side lens frame, and further, a step between the light receiving surface 5j of the dual purpose lens 5 and the resin cover with which the outer periphery of the rear side lens frame is covered, and in this case, a problem arises, that the mucus, dust and the like enter the inside of the field of view range of the dual purpose lens 5.

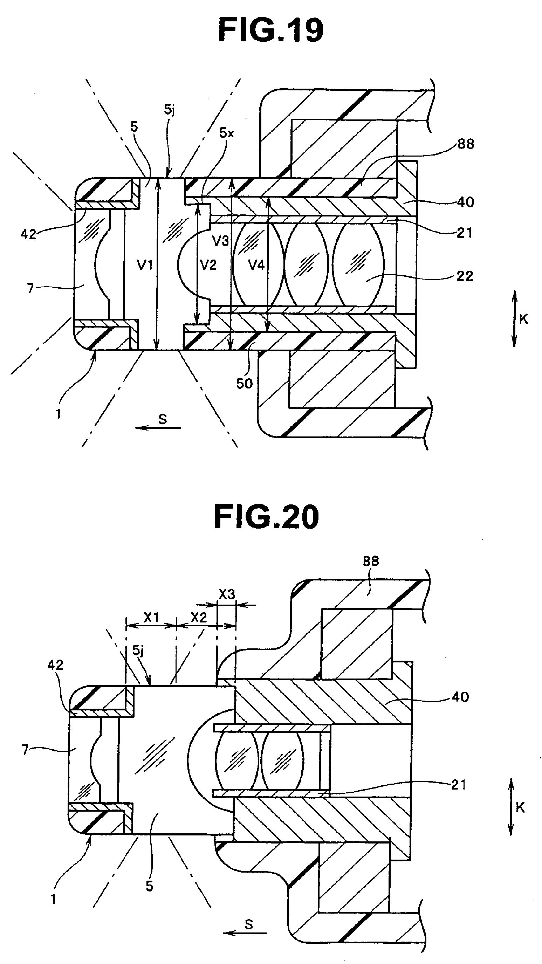

Hereinafter, configurations that solve the problem like these are shown with use of Figs. 19 and 20. Fig. 19 is a partial sectional view schematically showing a configuration in which a rear side lens frame and a resin cover is fixed to the proximal end side of the dual purpose lens without a step. Fig. 20 is a partial sectional view schematically showing a configuration in which an ineffective field of view area is provided at a light receiving surface of the dual purpose lens.

As shown in Fig. 19, the stepped portion 5x is formed at the rear side of the dual purpose lens 5, and the rear side lens frame 40 which holds the lens frame 21 which holds a plurality of lenses 22, and a resin cover 88 with which the outer periphery of the lens frame 40 is covered are fitted to the stepped portion 5x. An outer surface of the resin cover 88 and the light receiving surface 5j of the dual purpose lens 5 are at the same height.

More specifically, when an outside diameter in the diameter direction K of the dual purpose lens 5 is set to V1, an outside diameter in the diameter direction K of a region to which the rear side lens frame 40 is fitted, at the proximal end side of the dual purpose lens 5 is set to V2, an outside diameter in the diameter direction K of the resin cover 88 is set to V3, and an outside diameter in the diameter direction K of the rear side lens frame 21 is set to V4, V1 > V2 is satisfied, whereby the stepped portion 5x is formed, and V1 > V4 and V1 = V3 are satisfied, whereby the outer surface of the resin cover 88 becomes the surface having the same height as the light receiving surface 5j of the dual purpose lens 5.

Accordingly, a step does not occur between the light receiving surface 5j of the dual purpose lens 5 and the resin cover 88, and therefore, mucus, dust and the like do not accumulate in the step as described above.

Further, even if steps occur between the dual purpose lens 5, and the rear side lens frame 40 and the resin cover 88 after the rear side lens frame 40 and the resin cover 88 are fitted to the proximal end of the dual purpose lens 5 with a fitting length of X3, an effective field of view area X1 is provided at the distal end side of the light receiving surface 5j of the dual purpose lens 5, an ineffective field of view area X2 is provided at a proximal end side of the area X1 by painting the light receiving surface 5j in black or the like, and the rear side lens frame 40 is fitted to the dual purpose lens 5 so that the fitting length X3 becomes smaller in the insertion direction S than the area X2 as shown in Fig. 20. According to the configuration, even if mucus, dust and the like accumulate in the steps between the dual purpose lens 5, and the rear side lens frame 40 and the resin cover 88, the area in which the mucus, dust and the like accumulate is the ineffective field of view area X2, and therefore, mucus, dust and the like do not enter the field of view range of the dual purpose lens 5.

According to the configurations shown in Figs. 19 and 20 as above, a favorable field of view of the dual purpose lens 5 can be ensured.

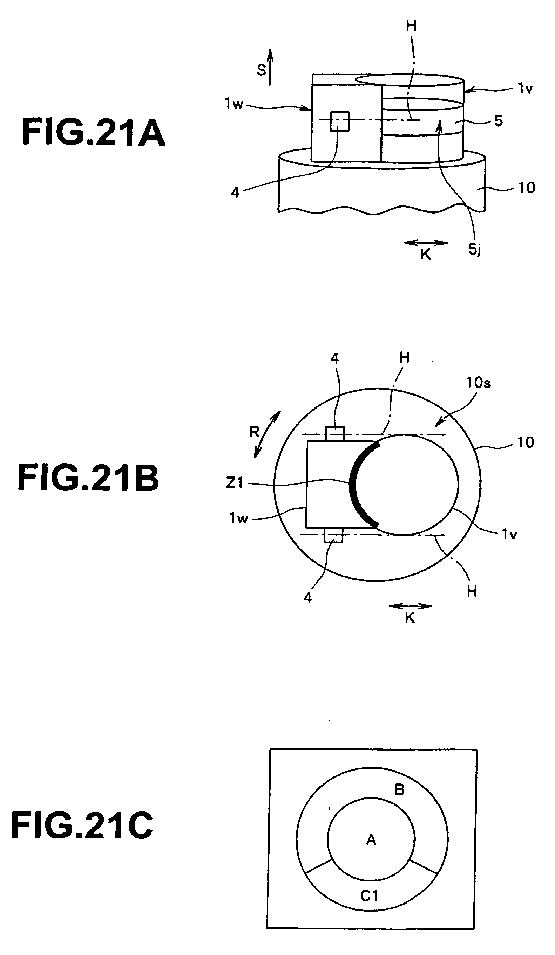

Fig. 21A is a perspective view showing a disposition location of a nozzle which supplies a fluid to a conventional dual purpose lens, at a distal end side of an insertion portion provided with the dual purpose lens. Fig. 21B is a top view of Fig. 21A. Fig. 21C is a view showing an observation image which is observed by the endoscope insertion portion of Fig. 21A.

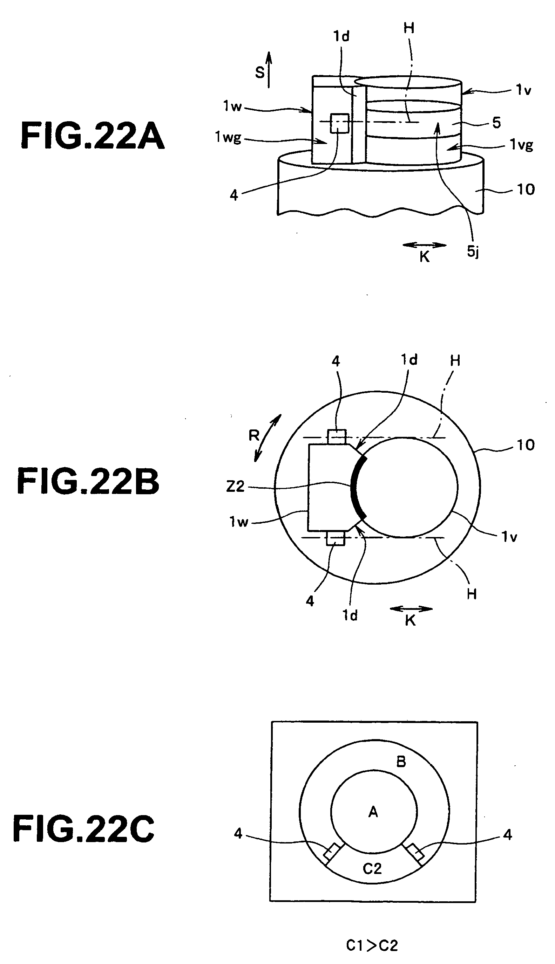

Further, Fig. 22A is a perspective view showing a disposition location of a nozzle in the case in which a recess is provided at a region between the outer peripheral side surface of the first projecting portion and the outer peripheral side surface of the second projecting portion, at a distal end side of the insertion portion provided with the dual purpose lens. Fig. 22B is a top view of Fig. 22A. Fig. 22C is a view showing an observation image which is observed by the endoscope insertion portion of Fig. 22A.

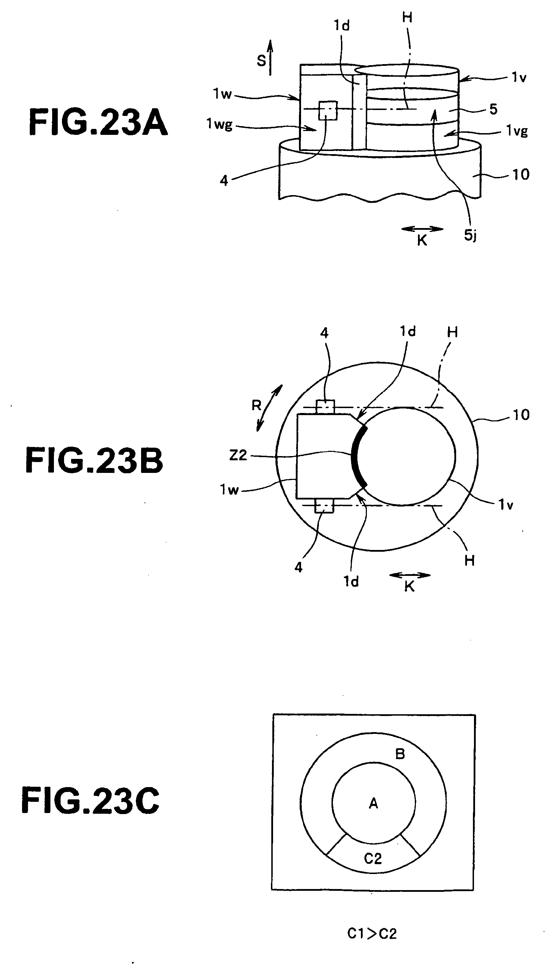

Further, Fig. 23A is a perspective view showing a disposition location of a nozzle in the case in which the nozzle is provided at an outer side in the diameter direction from Fig. 20 in the outer peripheral side surface of the second projecting portion, at the distal end side of the insertion portion where the dual purpose lens is provided. Fig. 23B is a top view of Fig. 23A. Fig. 23C is a view showing an observation image which is observed by the endoscope insertion portion of Fig. 23A.

Incidentally, in the endoscope 100 having the dual purpose lens 5 described above, the light receiving surface 5j of the dual purpose lens 5 is cleaned by a plurality of nozzles 4 which are provided on the outer peripheral side surface 1wg of the second projecting portion 1w, and supply a fluid to the light receiving surface 5j of the dual purpose lens 5.

However, when the nozzles 4 are provided at the locations along tangential lines H of the light receiving surface 5j of the dual purpose lens 5 in the diameter direction K in order to supply a fluid to the light receiving surface 5j of the dual purpose lens 5 reliably in the outer peripheral side surface 1wg of the second projecting portion 1w as shown in Figs. 21A and 21B, an area which hides the light receiving surface 5j of the dual purpose lens 5 becomes large in the second projecting portion 1w as shown by a thick line Z1 of Fig. 21B.

Here, as shown in Fig. 21C, the image of the second region to be examined which is picked up by the dual purpose lens 5 does not completely become annular on the monitor as shown by the area B, and only an area C1 which is hidden by the second projecting portion 1wa is not displayed.

Accordingly, when the area where the second projecting portion 1w hides the light receiving surface 5j of the dual purpose lens 5 becomes large, a problem arises, that the area C1 becomes large, that is, the area B becomes small, and the observation image of the second region to be examined becomes small.

Thus, as shown in Figs. 22A and 22B, a recess 1d is provided at a region where the outer peripheral side surface 1vg of the first projecting portion 1v and the outer peripheral side surface 1wg of the second projecting portion 1w are in contact with each other, in the diameter direction K, and as shown by a thick line Z2 of Fig. 22B, the area which hides the light receiving surface 5j of the dual purpose lens 5 in the second projecting portion 1w is made small. Thereby (Z2 < Z1), the configuration is conceivable, which makes an area C2 which is hidden by the second projecting portion 1w smaller than in the configuration of Fig. 21 in the observation image of the second region to be examined as shown in Fig. 22C even if the nozzles 4 are provided at the locations along the tangential lines H of the light receiving surface 5j of the dual purpose lens 5 in the diameter direction K (C2 < C1).

However, in the configuration of Fig. 22, a problem arises, that due to the recess 1d, the nozzles 4 are displayed on the observation image by the dual purpose lens 5, as shown in Fig. 22C.

Thus, as shown in Figs. 23A and 23B, the locations where the nozzles 4 are provided are shifted to an outer side in the diameter direction K from Fig. 20, in the outer peripheral side surface 1wg of the second projecting portion 1w while the recesses 1d are formed. More specifically, the nozzles 4 are disposed outside the observation range T2 of the dual purpose lens 5.

According to the configuration like this, the area which hides the light receiving surface 5j of the dual purpose lens 5 in the second projecting portion 1w can be made small as in Fig. 22 (Z2 < Z1), and even if the nozzles 4 are provided at the positions along the tangential lines H of the light receiving surface 5j of the dual purpose lens 5 in the diameter direction K, the area C2 which is hidden by the second projecting portion 1w can be made smaller than in the configuration of Fig. 21 in the observation image of the second region to be examined as shown in Fig. 23C (C2 < C1), and further, the nozzles 4 can be prevented from being on the observation image.

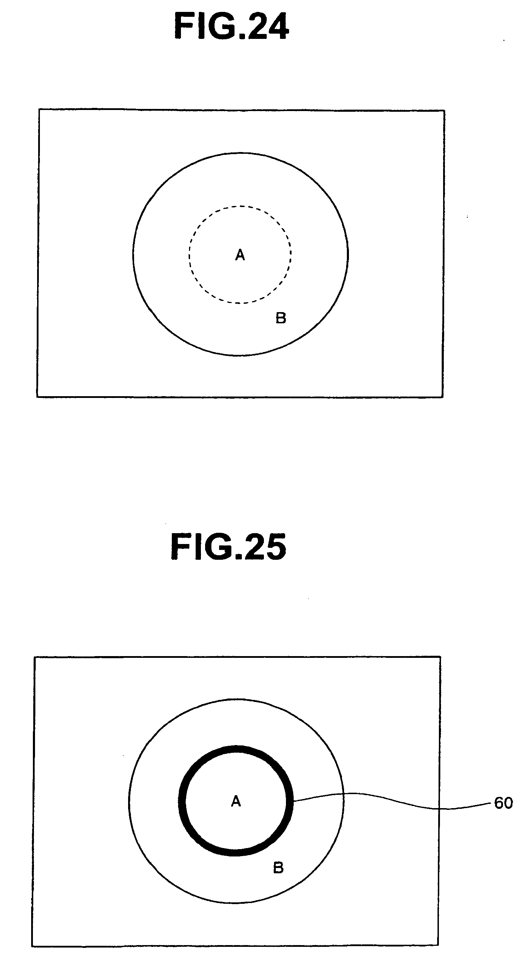

Fig. 24 is a view showing an observation image observed by the forward observing lens and the dual purpose lens.

Incidentally, as described above, the image observed by the forward observing lens 7 is displayed as the circular area A, and the image observed by the dual purpose lens 5 is displayed as the annular area B around the area A, as shown in Fig. 24. In Fig. 24, the area C which is hidden by the second projecting portion 1w is not illustrated.

However, the area B is displayed adjacently to the area A. Therefore, a problem arises, that it cannot be discriminated whether a specific area of interest which is displayed in the vicinity of the boundary of the area A and the area B is the image in the forward field of view observed by the forward observing lens 7, or the image in the circumferential direction field of view observed by the dual purpose lens 5, that is, the boundary of the area A and the area B cannot be discriminated, and it is difficult to determine whether the area of interest is located in the first region to be examined or located in the second region to be examined.

Accordingly, if the operator erroneously recognizes that the area of interest is located in the first region to be examined from the observation image when moving the dual purpose lens 5 close to the area of interest which is located in the second region to be examined, for example, the operator pushes and moves the insertion portion 93 forward in the insertion direction S to move the forward observing lens 7 close to the area of interest, and as a result, a problem arises, that the dual purpose lens 5 which is originally the object to be moved close is moved away from the area of interest.

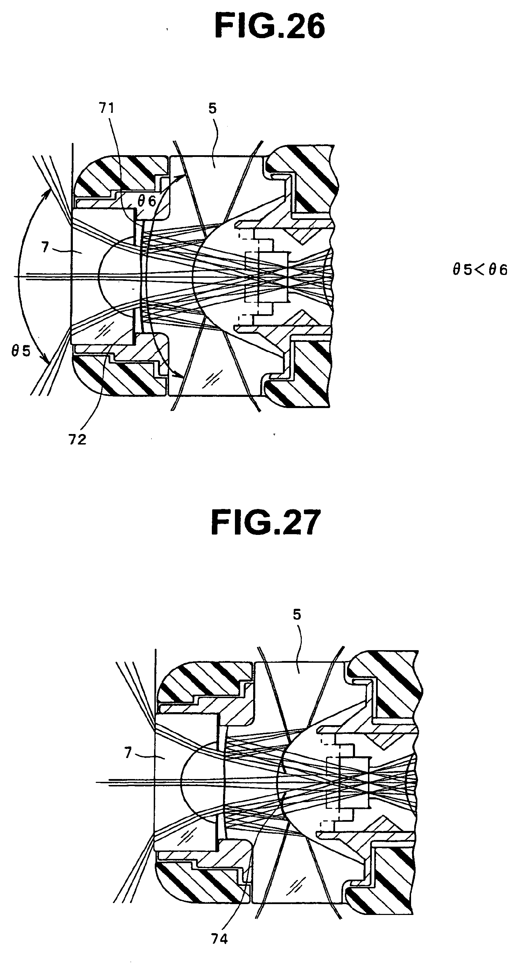

Hereinafter, configurations which solve the problem like this will be described with use of Figs. 25 to 28. Fig. 25 is a view showing an example in which a boundary line is provided between the area which displays the image picked up by the forward observing lens and the area which displays the image picked up by the dual purpose lens, of the observation image, and Fig. 26 is a partial sectional view of an insertion portion distal end side which shows a configuration of forming the boundary line by making a sideward viewing angle smaller than a forward viewing angle.

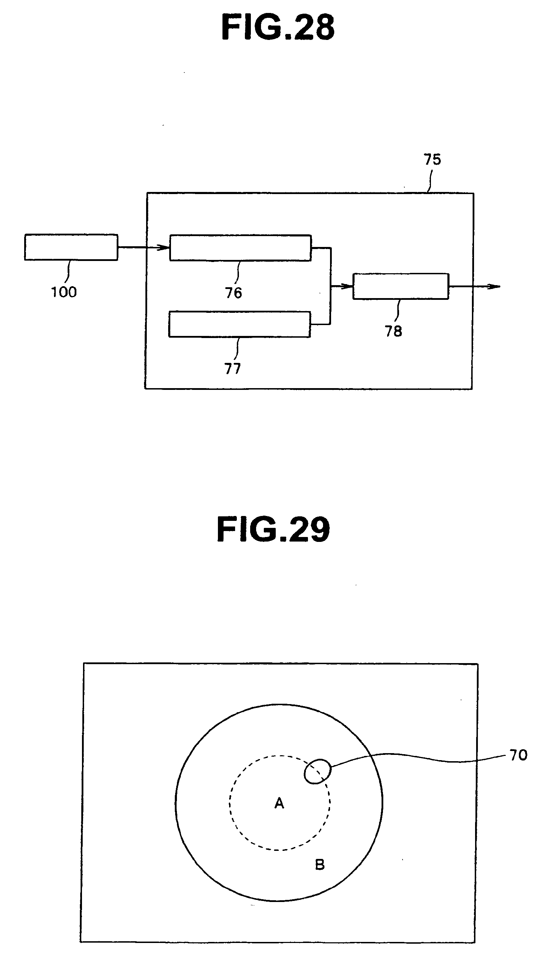

Further, Fig. 27 is a partial sectional view of an insertion portion distal end side showing a configuration of forming a boundary line by a mask, and Fig. 28 is a block diagram showing a configuration of electrically forming a boundary line in an observation image.

As shown in Fig. 26, a diaphragm 72 is usually provided on a proximal end surface of the forward observing lens 7, and a mirror coat 71 which reflects the light incident from the light receiving surface to the image pickup device 23 side is formed on the distal end surface of the dual purpose lens 5, and a forward viewing angle θ5 which is restricted by the diaphragm 72 is configured to be smaller than a sideward viewing angle θ6 which is restricted by an inside diameter of the mirror coat 71 (θ5 < θ6).

From the above, an area on which lights from the forward field of view and the circumferential direction field of view are not incident is formed for the image pickup device 23 by the diaphragm 72 and the mirror coat 71. The area is displayed as a deep-black boundary line 60 in the observation image as shown in Fig. 25.

According to the above, the operator can determine that the area of interest which is located at an inner side from the boundary line 60 is located in the first region to be examined, and can easily determine that the area of interest which is located at an outer side is located in the second region to be examined, from the observation image, and therefore, operability of the endoscope 100 is enhanced.

The boundary line 60 may be formed by providing a mask 74 on the proximal end surface of the dual purpose lens 5 as shown in Fig. 27.

Furthermore, the boundary line 60 may be formed by electrically superimposing the boundary line generated by boundary line generating means 77 on an endoscopic image which is outputted from endoscopic image generating means 76, which generates an endoscopic image picked up by the image pickup device 23, by superimposing means 78, in a known video processor 75 which is electrically connected to the light source apparatus to which the connector 96 of the endoscope 100 is connected.

Fig. 29 is a view showing a display example in which an area of interest is located across the area in which an image picked up by the forward observing lens is displayed and the area in which an image picked up by the dual purpose lens is displayed, in the observation image observed by the forward observing lens and the dual purpose lens.

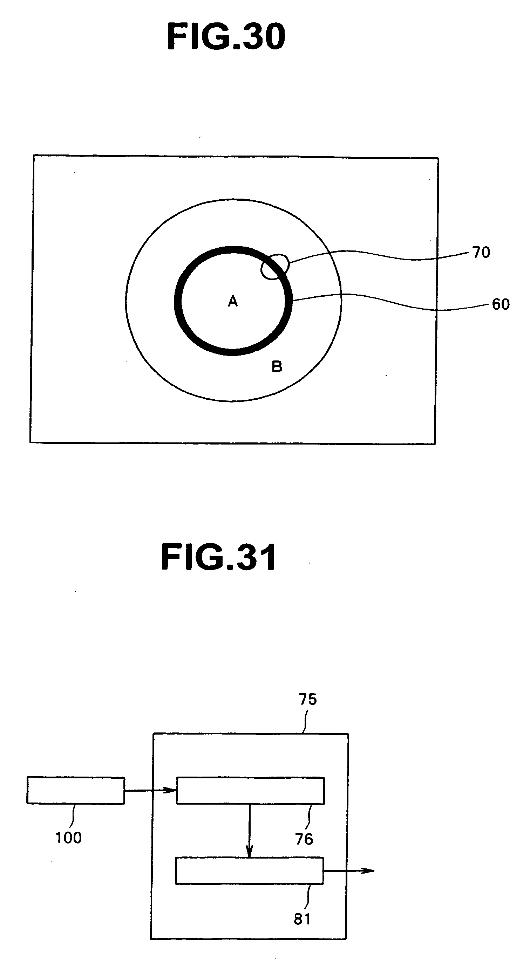

Further, Fig. 30 is a view showing a display example in which a boundary line is provided between the area which displays the image picked up by the forward observing lens and the area which displays the image picked up by the dual purpose lens, and the region of interest is located across the boundary line, in the observation image of Fig. 29.

Incidentally, as described above, the image observed by the forward observing lens 7 is displayed as the circular area A, and the image observed by the dual purpose lens 5 is displayed as the annular area B around the area A as shown in Fig. 29. In Fig. 29, illustration of the area C which is hidden by the second projecting portion 1w is also omitted.

However, the area B is displayed adjacently to the area A. Therefore, a problem arises, that it cannot be discriminated whether the specific area of interest which is displayed in the vicinity of the boundary of the area A and the area B is the image observed by the forward observing lens 7, or the image observed by the dual purpose lens 5, that is, the boundary of the area A and the area B cannot be discriminated, and it is difficult to determine whether the area of interest is located in the first region to be examined or located in the second region to be examined.

Thus, if the boundary line 60 is provided between the area A and the area B, as shown in Figs. 24 to 28 described above, a problem arises, that if the area of interest 70 is located across the area A and the area B as shown in Fig. 30, the area of interest 70 is divided by the boundary line 60 and observation of the area of interest 70 becomes difficult this time.

Hereinafter, a configuration which solves the problem like this will be described with use of the aforementioned Fig. 26 and Fig. 31. Fig. 31 is a block diagram showing a configuration which electrically deletes the boundary line from the observation image.

As described above, the forward viewing angle θ5 of the forward observing lens 7 is defined by the inside diameter of the diaphragm 72, whereas the sideward viewing angle θ6 of the dual purpose lens 5 is defined by the inside diameter of the mirror coat 71, and if the inside diameter of the mirror coat 71 is defined so that a reflection inside diameter of the circumferential direction field of view of reflection by the mirror coat 71 is smaller than the incident light diameter of the forward field of view defined by the diaphragm 72, that is, the inside diameter of the mirror coat 71 is made smaller than the inside diameter of the diaphragm 72, the circumferential direction field of view is superimposed on the forward field of view. Therefore, the boundary line 60 is not generated between the area A and the area B as shown in Fig. 29.

Accordingly, even if the area of interest 70 is located across the area A and the area B, observability of the area of interest 70 is not impaired.

As another configuration which deletes the boundary line 60, the configuration is conceivable, in which the insides diameters of the diaphragm 72 and the mirror coat 71 are set so that the incident light diameter of the forward field of view and the reflection inside diameter reflected by the mirror coat 71 correspond to each other, that is, the sideward viewing angle θ6 is superimposed onto the forward viewing angle θ5.

According to the above configuration, the circumferential direction field of view is superimposed onto the forward field of view, and therefore, the boundary line 60 is not generated between the area A and the area B as shown in Fig. 29.

Further, as another configuration that deletes the boundary line 60, the boundary line 60 may electrically be deleted by image regulating means 81 which is provided in the video processor 75, and matches the forward field of view image outer periphery and the circumferential direction field of view inner periphery with each other, as shown in Fig. 31.

The present application is filed claiming the priority of Japanese Patent Application No. 2010-156155 filed in Japan on July 8, 2010.

## Claims

1. An endoscope(100), comprising:
an insertion portion (93) that is inserted into a subject;
a projecting portion (1) that projects forward in an insertion direction (S)from a first distal end surface (10s) of a distal end portion (10) at a distal end side in the insertion direction of the insertion portion (93);
a forward observing lens (7) that is provided to face a second distal end surface (1ws) at a distal end side in the insertion direction (S) of the projecting portion (1), in the projecting portion (1), and observes a first region to be examined of the subject, which is located ahead in the insertion direction (S), of the second distal end surface (1ws);
a forward and sideward observing dual purpose lens (5) that is provided so that a light receiving surface (5j) faces an outer peripheral side surface (1vg) of the projecting portion (1) along the outer peripheral side surface of the projecting portion (1) at a rear side in the insertion direction (S) from the forward observing lens (7) in the projecting portion (1), and observes the first region to be examined with the forward observing lens (7);
**characterized by**
a first forward illuminating lens (12) that is provided to face the first distal end surface (10s), illuminates the first region to be examined, in the distal end portion (10), and fitted and located in a fit hole (10h) formed along the insertion direction (S) of the first distal end surface (10s);
a light shielding portion (20) which is located on a phantom line (Q) that linearly connects an end portion (5t) at a distal end side in the insertion direction (S), of the forward and sideward observing dual purpose lens (5) which faces the outer peripheral side surface (1vg), and an end portion (12at) at an outer side in a diameter direction (K) of the distal end portion (10) in the first forward illuminating lens (12), and which is constituted by an opening end portion (20m) located at an inner side in the diameter direction (K) of the fit hole (10h), and prevents illuminating light emitted from the first forward illuminating lens (12) from being incident on the light receiving surface (5j) of the forward and sideward observing dual purpose lens (5),
wherein the first distal end surface (10s) is formed to be an inclined surface that is inclined toward the first forward illuminating lens (12) from the projecting portion (1), and
wherein the opening end portion (20m) is located in an area outside an observation range (T2) of the forward and sideward observing dual purpose lens (5), and
wherein the forward and sideward observing dual purpose lens (5) observes, through the light receiving surface (5j), and within the observation range (T2), a second region to be examined of the subject, which is located opposite a circumference of the outer peripheral side surface (1vg).

2. The endoscope (100) according to claim 1, further comprising:
a second forward illuminating lens (2) that is provided to face the second distal end surface (1ws) in the projecting portion (1), and illuminates the first region to be examined.

3. The endoscope (100) according to claim 1 or 2, further comprising:
a sideward illuminating lens (6) that is provided to face the outer peripheral side surface (1vg) of the projecting portion (1) at a rear side in the insertion direction (S) from the forward and sideward observing dual purpose lens (5) in the projecting portion (1), and illuminates the second region to be examined,
wherein the sideward illuminating lens (6) is fixed to a lens frame, and the light shielding portion (20m) is configured at the lens frame.

4. The endoscope (100) according to claim 3,
wherein the first forward illuminating lens (12) is formed integrally with the sideward illuminating lens (6).

5. The endoscope (100) according to claim 4, further comprising one
light guide (34) for supplying illuminating light to the first forward illuminating lens (12), and the sideward illuminating lens (6).

6. The endoscope (100) according to claim 1,
wherein the first forward illuminating lens (12) comprises a first lens (12a) that faces the first distal end surface (10s), and a second lens (12b) and
wherein the second lens (12b) is disposed to be offset to an inner side in the diameter direction (K) of the distal end portion (10), with respect to the first lens (12a) such that an illumination light emitted from the second lens (12b) is refracted in a proximal end surface (12ab) of the first lens (12a).

## Patentansprüche

1. Endoskop (100) mit:
einem Einführbereich (93), der in einen Patienten eingeführt wird;
einem Vorsprungteil (1), das in einer Einführrichtung (S) von einer ersten distalen Endfläche (10s) eines distalen Endbereichs (10) an einer distalen Endseite in der Einführrichtung des Einführbereichs (93) nach vorne vorspringt;
einer nach vorne gerichteten Beobachtungslinse (7), die so in dem Vorsprungteil (1) vorgesehen ist, dass sie einer zweiten distalen Endfläche (1ws) an einer distalen Endseite in der Einführrichtung (S) des Vorsprungteils (1) zugewandt ist, und eine erste zu prüfende Region des Patienten beobachtet, die in der Einführrichtung (S) vor der zweiten distalen Endfläche (1ws) liegt;
einer nach vorne und zur Seite gerichteten Doppelfunktion-Beobachtungslinse (5), die so vorgesehen ist, dass eine Lichteingangsfläche (5j) einer Außenumfang-Seitenfläche (1vg) des Vorsprungteils (1) entlang der Außenumfang-Seitenfläche des Vorsprungteils (1) an einer Rückseite in der Einführrichtung (S) von der nach vorne gerichteten Beobachtungslinse (7) im Vorsprungteil (1) zugewandt ist, und die erste zu prüfende Region mit der nach vorne gerichteten Beobachtungslinse (7) beobachtet;
**gekennzeichnet durch**
eine erste nach vorne gerichtete Beleuchtungslinse (12), die so im distalen Endbereich (10) vorgesehen ist, dass sie der ersten distalen Endfläche (10s) zugewandt ist, die erste zu prüfende Region beleuchtet, und in einem Passloch (10h) der ersten distalen Endfläche (10s) eingepasst und angeordnet ist, welches entlang der Einführrichtung (S) ausgebildet ist;
ein Lichtabschirmungsteil (20), das auf einer fiktiven Linie (Q) angeordnet ist, die einen der Außenumfang-Seitenfläche (1vg) zugewandten Endbereich (5t) an einer in der Einführrichtung (S) distalen Endseite der nach vorne und zur Seite gerichteten Doppelfunktion-Beobachtungslinse (5) und einen Endbereich (12at) an einer Außenseite in einer Durchmesserrichtung (K) des distalen Endbereichs (10) in der ersten nach vorne gerichteten Beleuchtungslinse (12) linear verbindet, und welches **durch** einen Öffnungsendbereich (20m) gebildet ist, der an einer Innenseite in der Durchmesserrichtung (K) des Passlochs (10h) angeordnet ist, und welches verhindert, dass von der ersten nach vorne gerichteten Beleuchtungslinse (12) ausgesendetes Beleuchtungslicht auf die Lichteingangsfläche (5j) der nach vorne und zur Seite gerichteten Doppelfunktion-Beobachtungslinse (5) einfällt,
wobei die erste distale Endfläche (10s) als eine geneigte Fläche ausgebildet ist, die vom Vorsprungteil (1) zu der ersten nach vorne gerichteten Beleuchtungslinse (12) hin geneigt ist, und
wobei der Öffnungsendbereich (20m) in einem Bereich außerhalb eines Beobachtungsraums (T2) der nach vorne und zur Seite gerichteten Doppelfunktion-Beobachtungslinse (5) angeordnet ist, und
wobei die nach vorne und zur Seite gerichtete Doppelfunktion-Beobachtungslinse (5) **durch** die Lichteingangsfläche (5j) und innerhalb des Beobachtungsraumes (T2) eine zweite zu prüfende Region des Patienten beobachtet, die gegenüber einem Umfang der Außenumfang-Seitenfläche (1vg) liegt.

2. Endoskop (100) nach Anspruch 1, das des Weiteren umfasst:
eine zweite nach vorne gerichtete Beleuchtungslinse (2), die so vorgesehen ist, dass sie der zweiten distalen Endfläche (1ws) im Vorsprungteil (1) zugewandt ist, und die die erste zu prüfende Region beleuchtet.

3. Endoskop (100) nach Anspruch 1 oder 2, das des Weiteren umfasst:
eine zur Seite gerichtete Beleuchtungslinse (6), die so im Vorsprungteil (1) vorgesehen ist, dass sie der Außenumfang-Seitenfläche (1vg) des Vorsprungteils (1) auf einer Rückseite in der Einführrichtung (S) von der nach vorne und zur Seite gerichteten Doppelfunktion-Beobachtungslinse (5) zugewandt ist, und die die zweite zu prüfende Region beleuchtet,
wobei die zur Seite gerichtete Beleuchtungslinse (6) an einem Linsenrahmen befestigt ist und das Lichtabschirmungsteil (20m) an dem Linsenrahmen ausgebildet ist.

4. Endoskop (100) nach Anspruch 3,
wobei die erste nach vorne gerichtete Beleuchtungslinse (12) einstückig mit der zur Seite gerichteten Beleuchtungslinse (6) ausgebildet ist.

5. Endoskop (100) nach Anspruch 4, das des Weiteren umfasst
einen Lichtleiter (34) zum Zuführen von Beleuchtungslicht zu der ersten nach vorne gerichteten Beleuchtungslinse (12) und der zur Seite gerichteten Beleuchtungslinse (6).

6. Endoskop (100) nach Anspruch 1,
wobei die erste nach vorne gerichtete Beleuchtungslinse (12) eine erste Linse (12a), die der ersten distalen Endfläche (10s) zugewandt ist, und eine zweite Linse (12b) aufweist und
wobei die zweite Linse (12b) so angeordnet ist, dass sie in Bezug auf die erste Linse (12a) zu einer Innenseite in der Durchmesserrichtung (K) des distalen Endbereichs (10) so versetzt ist, dass ein von der zweiten Linse (12b) ausgesendetes Beleuchtungslicht in einer proximalen Endfläche (12ab) der ersten Linse (12a) gebrochen wird.

## Revendications

1. Endoscope (100) comprenant :
une partie (93) d'insertion qui est insérée dans un sujet ;
une partie (1) de projection qui se projette vers l'avant dans une direction d'insertion (S) depuis une première surface (10s) d'extrémité distale d'une partie (10) d'extrémité distale au niveau d'un côté d'extrémité distale dans la direction d'insertion de la partie (93) d'insertion ;
une lentille (7) d'observation vers l'avant qui est prévue pour être en regard d'une deuxième surface (1ws) d'extrémité distale au niveau d'un côté d'extrémité distale dans la direction d'insertion (S) de la partie (1) de projection, dans la partie (1) de projection, et observe une première région devant être examinée du sujet, qui est située à l'avant dans la direction d'insertion (S), de la deuxième surface (1ws) d'extrémité distale ;
une lentille (5) à double utilisation d'observation vers l'avant et sur les côtés qui est prévue de sorte qu'une surface (5j) de réception de lumière est en regard d'une surface (1vg) latérale périphérique externe de la partie (1) de projection le long de la surface latérale périphérique externe de la partie (1) de projection au niveau d'un côté arrière dans la direction d'insertion (S) depuis la lentille (7) d'observation vers l'avant dans la partie (1) de projection, et observe la première région devant être examinée avec la lentille (7) d'observation vers l'avant ;
**caractérisé par**
une première lentille (12) d'éclairage vers l'avant qui est prévue pour être en regard de la première surface (10s) d'extrémité distale, éclaire la première région devant être examinée, dans la partie (10) d'extrémité distale, et ajustée et placée dans un trou (10h) d'ajustement formé le long de la direction d'insertion (S) de la première surface (10s) d'extrémité distale ;
une partie (20) de protection de lumière qui est placée sur une ligne fantôme (Q) qui connecte de manière linéaire une partie (5t) d'extrémité au niveau d'un côté d'extrémité distale dans la direction d'insertion (S), de la lentille (5) à double utilisation d'observation vers l'avant et sur les côtés qui est en regard de la surface (1vg) latérale périphérique externe, et une partie (12at) d'extrémité au niveau d'un côté externe dans une direction de diamètre (K) de la partie (10) d'extrémité distale dans la première lentille (12) d'éclairage vers l'avant, et qui est constituée par une partie (20m) d'extrémité d'ouverture placée au niveau d'un côté interne dans la direction de diamètre (K) du trou (10h) d'ajustement, et empêche la lumière d'éclairage émise depuis la première lentille (12) d'éclairage vers l'avant d'être incidente sur la surface (5j) de réception de lumière de la lentille (5) à double utilisation d'observation vers l'avant et sur les côtés,
dans lequel la première surface (10s) d'extrémité distale est formée pour être une surface inclinée qui est inclinée vers la première lentille (12) d'éclairage vers l'avant depuis la partie (1) de projection, et
dans lequel la partie (20m) d'extrémité d'ouverture est placée dans une zone à l'extérieur d'une plage d'observation (T2) de la lentille (5) à double utilisation d'observation vers l'avant et sur les côtés, et
dans lequel la lentille (5) à double utilisation d'observation vers l'avant et sur les côtés observe, à travers la surface (5j) de réception de lumière, et à l'intérieur de la plage d'observation (T2), une deuxième région devant être examinée du sujet, qui est placée opposée à une circonférence de la surface (1vg) latérale périphérique externe.

2. Endoscope (100) selon la revendication 1, comprenant en outre :
une deuxième lentille (2) d'éclairage vers l'avant qui est prévue pour être en regard de la deuxième surface (1ws) d'extrémité distale dans la partie (1) de projection, et éclaire la première région devant être examinée.

3. Endoscope (100) selon la revendication 1 ou 2, comprenant en outre :
une lentille (6) d'éclairage vers les côtés qui est prévue pour être en regard de la surface (1vg) latérale périphérique externe de la partie (1) de projection au niveau d'un côté arrière dans la direction d'insertion (S) depuis la lentille (5) à double utilisation d'observation vers l'avant et sur les côtés dans la partie (1) de projection, et éclaire la deuxième région devant être examinée,
dans lequel la lentille (6) d'éclairage sur les côtés est fixée à une monture de lentille, et la partie (20m) de protection de lumière est configurée au niveau de la monture de lentille.

4. Endoscope (100) selon la revendication 3,
dans lequel la première lentille (12) d'éclairage vers l'avant est formée solidairement avec la lentille (6) d'éclairage sur les côtés.

5. Endoscope (100) selon la revendication 4, comprenant en outre
un guide de lumière (34) destiné à délivrer une lumière d'éclairage vers la première lentille (12) d'éclairage vers l'avant, et la lentille (6) d'éclairage sur les côtés.

6. Endoscope (100) selon la revendication 1,
dans lequel la première lentille (12) d'éclairage vers l'avant comprend une première lentille (12a) qui est en regard de la première surface (10s) d'extrémité distale, et une deuxième lentille (12b) et
dans lequel la deuxième lentille (12b) est disposée pour être décalée vers un côté interne dans la direction de diamètre (K) de la partie (10) d'extrémité distale, par rapport à la première lentille (12a) d'une manière telle qu'une lumière d'éclairage émise depuis la deuxième lentille (12b) est réfractée dans une surface d'extrémité proximale (12ab) de la première lentille (12a).
